(19) **Europäisches Patentamt**
**European Patent Office**
**Office européen des brevets**

(11) **EP 4 620 937 A1**

(12) **EUROPEAN PATENT APPLICATION**

(43) Date of publication:
24.09.2025 Bulletin 2025/39

(21) Application number: 24165283.3

(22) Date of filing: **21.03.2024**

(51) International Patent Classification (IPC):
*C07C 2/78* (2006.01)  *C07C 11/24* (2006.01)
*C01B 3/36* (2006.01)  *C25B 1/04* (2021.01)

(52) Cooperative Patent Classification (CPC):
(C-Sets available)
**C07C 2/78; C01B 3/36; C25B 1/04;** C01B 2203/061
(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: BASF SE
**67056 Ludwigshafen am Rhein (DE)**

(72) Inventors:
• **KRUEGER, Marco**
**67056 Ludwigshafen am Rhein (DE)**

• **HUEFFER, Stephan**
**67056 Ludwigshafen am Rhein (DE)**
• **WEICHERT, Christian**
**67056 Ludwigshafen am Rhein (DE)**
• **SENTKO, Matthias Martin**
**67056 Ludwigshafen am Rhein (DE)**

(74) Representative: **Schuck, Alexander
Patentanwälte
Isenbruck Bösl Hörschler PartG mbB
Eastsite One
Seckenheimer Landstraße 4
68163 Mannheim (DE)**

(54) **ELECTROLYSIS OXYGEN FOR SUSTAINABLE ACETYLENE CHEMISTRY**

(57) A process for preparing acetylene and/or synthesis gas by partial oxidation of hydrocarbons with an oxidizing agent, wherein the oxidizing agent comprises $O_2$ and $H_2$, wherein the oxidizing agent is obtained at least in part by water splitting, preferably by electrolysis, the water splitting, preferably the electrolysis, preferably using energy generated at least in part from non-fossil resources, a cracking gas stream obtainable by the process according to the present invention, acetylene obtainable by the process according to the present invention, acetylene having a low total cradle to gate product carbon footprint, synthesis gas obtainable by the process according to the present invention, synthesis gas comprising hydrogen, CO, $CO_2$ and $CH_4$, wherein the separated synthesis gas stream has a $\delta^{18}O$ value of < 22 %o, referred to the international standard VSMOW ((Vienna-Standard- Mean-Ocean- Water)), the use of an oxidizing agent comprising $O_2$ and $H_2$ for the preparation of acetylene and synthesis gas, the use of the inventive acetylene or the acetylene obtained by the inventive process for the preparation of butynediol, butanediol, butenediol, polybutylene terephthalate (PBT), polybutylene adipate terephthalate (PBAT), tetrahydrofurane (THF), polytetrahydrofurane (polyTHF), polyester-based thermoplastic polyurethanes (TPUs), polyether-based TPUs, gamma-butyrolactone, pyrrolidine, vinylpyrrolidone, polyvinylpyrrolidone, N-methylpyrrolidone, vinyl ether, polyvinyl ether, terpenes and downstream products thereof, and in welding processes and cutting of metals; a process for preparing 1 ,4-butanediol wherein at least in part the inventive acetylene or the acetylene obtained by the inventive process is employed; the use of the inventive synthesis gas and the synthesis gas obtained according to the process of the present invention for the preparation of methanol, fuel applications, formaldehyde, acetic acid, olefins, sodium methylate, methylation products, dimethylterephthalate, methylamine, methyl mercaptane and downstream products; a process for preparing methanol from a synthesis gas wherein the synthesis gas is at least in part the synthesis gas the inventive synthesis gas or the synthesis gas obtained by the inventive process; and a process for preparing formaldehyde from a synthesis gas comprising the oxidation of the methanol obtained in the inventive process to formaldehyde with oxygen, air or a mixture thereof in the presence of a catalyst.

EP 4 620 937 A1

(52)  Cooperative Patent Classification (CPC): (Cont.)

C-Sets
**C07C 2/78, C07C 11/24**

**Description**

The present invention relates to a process for preparing acetylene and/or synthesis gas by partial oxidation of hydrocarbons with an oxidizing agent, wherein the oxidizing agent comprises $O_2$ and $H_2$, wherein the oxidizing agent is obtained at least in part by water splitting, preferably by electrolysis, the water splitting, preferably the electrolysis, preferably using energy generated at least in part from non-fossil resources, a cracking gas stream obtainable by the process according to the present invention, acetylene obtainable by the process according to the present invention, acetylene having a low total cradle to gate product carbon footprint, synthesis gas obtainable by the process according to the present invention, synthesis gas comprising hydrogen, CO, $CO_2$ and $CH_4$, wherein the separated synthesis gas stream has a $\delta^{80}O$ value of < 22 ‰o, referred to the international standard VSMOW (Vienna- Standard- Mean-Ocean-Water), the use of an oxidizing agent comprising $O_2$ and $H_2$ for the preparation of acetylene and synthesis gas, the use of the inventive acetylene or the acetylene obtained by the inventive process for the preparation of butynediol, butanediol, butenediol, polybutylene terephthalate (PBT), polybutylene adipate terephthalate (PBAT), tetrahydrofurane (THF), polytetrahydrofurane (polyTHF), polyester-based thermoplastic polyurethanes (TPUs), polyether-based TPUs, gamma-butyrolactone, pyrrolidine, vinylpyrrolidone, polyvinylpyrrolidone, N-methylpyrrolidone, vinyl ether, polyvinyl ether, terpenes and downstream products thereof; butynediol, butanediol, butenediol, polybutylene terephthalate (PBT), polybutylene adipate terephthalate (PBAT), tetrahydrofurane (THF), polytetrahydrofurane (polyTHF), polyester-based thermoplastic polyurethanes (TPUs), polyether-based TPUs, gamma-butyrolactone, pyrrolidine, vinylpyrrolidone, polyvinylpyrrolidone, N-methylpyrrolidone, vinyl ether, polyvinyl ether, terpenes and downstream products thereof obtained from the acetylene according to the present invention or obtained from the acetylene obtained by the process according to the present invention; and in welding processes and cutting of metals, a process for preparing 1 ,4-butanediol wherein at least in part the inventive acetylene or the acetylene obtained by the inventive process is employed; the use of the inventive synthesis gas and the synthesis gas obtained according to the process of the present invention for the preparation of methanol, fuel applications, formaldehyde, acetic acid, olefins, sodium methylate, methylation products, dimethylterephthalate, methylamine, methyl mercaptane and downstream products; a process for preparing methanol from a synthesis gas wherein the synthesis gas is at least in part the inventive synthesis gas or the synthesis gas obtained by the inventive process; a process for preparing formaldehyde from a synthesis gas comprising the oxidation of the methanol obtained in the inventive process to formaldehyde with oxygen, air or a mixture thereof in the presence of a catalyst.

The key commercial routes to produce acetylene are characterized by the temperature range of the cracking reactions of the selected hydrocarbon feed, typically in the range of 1400 to 1700 °C. The focus of the analysis in this report will be the production of acetylene via the processes listed below:
Partial oxidation of light hydrocarbons.

Steam cracker acetylene: 1 to 2 percent of acetylene, related to the severity of operating conditions, is produced in steam crackers. The acetylene can be hydrogenated to ethylene or recovered by separation using a solvent.

Electric arc or plasma arc: Cracking of light hydrocarbons in an electric arc to produce acetylene.

Calcium carbide: Acetylene is generated by the reaction of calcium carbide and water.

The idea of the partial oxidation process is to burn part of the hydrocarbons, e.g. methane as fuel to provide the energy required for the conversion of light hydrocarbons to acetylene. An important partial oxidation process is known as the Sachsse-Bartholomé Process (BASF). Various other industrial acetylene synthesis technologies based on the same partial oxidation process are known for example as Montecatini Process, the SBA Process (Société belge de l'Azote), the Wulff process and the Hoechst high-temperature pyrolysis process (HTP) differing for example in details of the burner as well as downstream quench and acetylene recovery designs. Instead of using a single burner also two stage burners in sequence can be used. Multiple production lines with parallel burner configuration are commonly used as well.

The partial oxidation is a high-temperature reaction which is typically conducted in a reactor system comprising a mixing unit, a burner block and a quench unit, and is described, for example, in Ullmann's Encyclopedia of Industrial Chemistry, 2012 Wiley-VCH Verlag GmbH & Co. KGaA, Weinheim, "Acetylene") and Gannon, R.E. et al, "Acetylene from Hydrocarbons," in Kirk-Othmer Encyclopedia of Chemical Technology, Volume 1, John Wiley & Sons Inc., 2000, pp. 177-227. According to in Ullmann's Encyclopedia of Industrial Chemistry, 2012 Wiley-VCH Verlag GmbH & Co. KGaA, Weinheim, "Acetylene", the feedstocks are continuously heated separately in preheaters. The heated feedstocks are mixed in a mixing unit and supplied via a mixing diffuser to a burner and further to a combustion chamber. Downstream of the combustion chamber, nozzles are used to supply an aqueous quench medium to the cracking gas, which is cooled rapidly to about 80-130°C. Through suitable selection of the oxygen ratio λ (λ<0.31), the open water-quench process was conducted such that the yield of acetylene based on the dry cracking gas is at an optimum (>8%). In this context, oxygen ratio λ is understood to mean the ratio of the amount of oxygen actually present to the stoichiometrically necessary amount of oxygen required for the full combustion of the feedstocks. At the same time, the soot loading of the cracking gas is also at a maximum.

Clearly, the soot produced in the partial oxidation process is an unwanted by-product leading to difficulties in processing, cleaning and waste disposal operations. High soot generation of an open water-quench acetylene process is accompanied with various emission sources which do not comply with most current environmental standards any more.

There are many attempts to reduce the soot formation in the partial oxidation process for acetylene synthesis.

A process for preparing acetylene and synthesis gas by partial oxidation of hydrocarbons with oxygen is described in US 5,824,834. This is a closed water quench process which is optimized for low soot volumes and is operated with a lean feed stream, specifically with a feed stream having an oxygen ratio λ>0.31. By using such higher oxygen ratio, the generation of soot and other unwanted by-products can be reduced so that current emission standards for a closed water quench acetylene process can be fulfilled. However, the closed water-quench process has the disadvantage of a reduced yield of acetylene value product.

A further process for preparing acetylene and synthesis gas by partial oxidation of hydrocarbons with oxygen is described in EP-A 12171956. This is a process which combines the advantages of the two above-described processes, i.e. optimized yield of acetylene product of value according to Ullmann's Encyclopedia of Industrial Chemistry, 2012 Wiley-VCH Verlag GmbH & Co. KGaA, Weinheim, "Acetylene", and compliance with applicable environmental protection regulations in accordance with US 5,824,834.

A further process for preparing acetylene and synthesis gas by partial oxidation of hydrocarbons with oxygen is described in EP-A 1 989 160. This is an extension of the process of US 5,824,834, such that the remaining very fine solid components (soot, tar, coke) in the product gas are separated out by means of liquid injection into product gas compressors.

US 9,802,875 relates to a reactor for preparing acetylene and synthesis gas by partial oxidation of hydrocarbons with oxygen. The reactor has a burner block with a firing space for acetylene preparation, a secondary space formed within the burner block, and an annular space surrounding the secondary space. The burner block has holes for supply of a stream of a mixture of hydrocarbons and oxygen to the firing space and holes for supply of a stream of auxiliary oxygen to the firing space. The holes for supply of a stream of auxiliary oxygen to the firing space are connected to the secondary space. The secondary space is connected to the annular space. The basic idea of US 9,802,875 is to no longer supply the auxiliary oxygen via individual lines, but instead to introduce it into the reaction space by means of a separate distributor ring via an intermediate space through individual holes or channels, the intermediate space being designed, in terms of flow mechanics, such that it ensures homogeneous distribution over the holes that it supplies.

US 9,580,312 relates to a continuous process for preparing acetylene and synthesis gas by partial oxidation of hydrocarbons with oxygen, in which a first input stream comprising one or more hydrocarbons and a second input stream comprising oxygen

- are separately heated in preheaters, mixed in a mixing unit in a ratio of the mass flow of the second input stream to the first input stream corresponding to an oxygen ratio of less than or equal to 0.31, the oxygen ratio being understood to mean the ratio of the amount of oxygen actually present in the second input stream to the amount of oxygen which is needed stoichiometrically and is required for the full combustion of the one or more hydrocarbons present in the first input stream,
- supplied via a burner block to a combustion chamber in which the partial oxidation of the hydrocarbons takes place,
- to obtain a first cracking gas stream Ig , wherein
- the first cracking gas stream Ig is cooled in a prequench by injection of an aqueous quench medium to a temperature in the range from 100 to 1000° C. to obtain a second cracking gas stream IIg ,
- in a solid-gas separation apparatus, 50 to 90% of the solids present in the second cracking gas stream IIg are removed therefrom to obtain a solids stream If and a third cracking gas stream IIIg,
- the third cracking gas stream IIIg is cooled in a total quench by injection of water to 80 to 90° C. to obtain a fourth cracking gas stream IVg and a first process water stream IIliq ,
- the fourth cracking gas stream IVg is subjected in one or more scrubbing apparatuses to a fine removal of solids to obtain one or more process water streams IIIliq , IIIIliq and a product gas stream VIg,
- the process water streams IIliq, IIIliq, IIIIliq are combined to give a combined process water stream IVIliq,
- the combined process water stream IVIliq is recycled partly as stream Vliq into the total quench and the rest is subjected as stream VIIliq to cleaning by partial vaporization in a one-stage flash vessel, stream VIIliq being vaporized in a proportion of 0.01 to 10% by weight, based on the total weight thereof, to obtain a cleaned process water stream VIIIliq which is cooled by means of a re-cooling device, partly recycled as stream VIIIliq into one or more of the one or more scrubbing apparatuses, and the rest is discharged as stream IXliq , and supplied to the wastewater in need of treatment.

It has been found according to US 9,580,312 that the specific process regime, through pre-quenching of the cracking gas stream from a process for continuously preparing acetylene and synthesis gas to a temperature in the range from 100 to 1000° C, avoids both unwanted condensation of water of reaction, quench water or tars prior to supply of the cracking gas stream obtained here to a downstream solid-gas separation apparatus, and thermal overloading of the solid-gas separation apparatus, and can additionally ensure stoppage of the synthesis process and hence an optimal acetylene yield.

However, the above prior art mainly mentions ways of separating the soot obtained in the acetylene synthesis by partial oxidation, but does not give any pointer to the problem of the increased occurrence of soot, coke and tar, and how these can be further actively prevented and reduced/avoided without compromising the acetylene yield.

All processes for the production of acetylene directly from methane through partial oxidation mentioned above use exclusively conventional oxygen gas as oxidant in the partial oxidation process.

The oxygen employed in the partial oxidation process for preparing acetylene is generally obtained by separation from the atmosphere. Commercial large-scale air separation plants (ASU, Air Separation Unit) are based on cryogenic distillation technology, capable of supplying oxygen at high purity and pressure. The conventional oxygen obtained by separation from the atmosphere generally comprises a certain amount of air (i.e. mainly $N_2$, Argon, $CO_2$) e.g. Oxygen 2.5 comprises 99.5 Vol% $O_2$ and $\leq 5000$ ppmv air. Oxygen 2.5 is a typical "technical grade" oxygen gas used for industrial partial oxidation processes. However, Oxygen ($O_2$) production, especially the production of high purity oxygen, is a major part of the energy consumption and capital cost of for industrial processes such as partial oxidation requiring large quantities of oxygen as a raw material. Typical electric power consumption for conventional ASU plants is approx. 245 kWh/t of oxygen 99% purity (Linde Engineering Group, see Hesari et al, General design consideration of cryogenic air separation unit for esfahan steel company, J. Gas Technology, Vol. 5, Issue 1 (2020), pp. 32-42) and this energy consumption is increasing steeply with even higher oxygen purity requirements such as Oxygen 2.5 and above (doi: 10.1016/j.egypro.2014.11.054, Fig. 4)

A relevant part of the total (cradle to gate) product carbon footprint of acetylene prepared by the partial oxidation process is therefore based on the raw material oxygen employed.

It was accordingly an object of the invention to provide a process for preparing acetylene and/or synthesis gas by partial oxidation of hydrocarbons which combines reduction/avoidance of unwanted by-products (tar, coke, soot) at high acetylene yield with environmental friendliness characterized by a low PCF of acetylene and synthesis gas products.

The reduction of greenhouse gas emissions and therefore the reduction of the product carbon footprint is one of the main issues of our time.

A way of reducing the product carbon footprint of acetylene and synthesis gas is the provision of a high-yield process with a total raw material and energy consumption which is as low as possible, the utilization of raw materials and energy with a low carbon emission backpack as well as the avoidance of waste generation.

Hydrogen is considered a promising candidate that is increasingly intended to replace fossil fuels. To cut down greenhouse gas emissions, it is essential that future hydrogen production is based on renewable energy sources. One such possibility is the electrolysis of water, provided it is operated with electricity from renewable energy sources. Oxygen is produced as a by-produc-tof water electrolysis, which is usually released into the environment without further use. The electrolysis of 9 tons of water gives 1 ton of hydrogen and 8 tons of oxygen gas, but usually only the hydrogen is used as a value product.

The use of the by-product oxygen from electrolysis in the process for preparing acetylene and/or synthesis gas by partial oxidation of hydrocarbons could improve the overall efficiency of the water electrolysis process.

Additionally, the environmental friendliness of the process for preparing acetylene and/or synthesis gas by partial oxidation of hydrocarbons could be improved, since the energy demanding provision of the oxygen obtained by conventional air separation from the atmosphere generally used in said process could be replaced by the provision of surplus oxygen from the electrolysis of water. This is especially relevant for green hydrogen production via water electrolysis based on renewable energy because in this case the oxygen by-product also automatically benefits from the allocation of the scarce renewable energy leading to low PCF oxygen production without any additional consumption of renewable energy.

However, the by-product oxygen from electrolysis comprises not negligible quantities of hydrogen and water. Residual hydrogen is always found in the oxygen gas due to cross-over effects of the very mobile $H_2$ molecules and the gaseous electrolysis products show close to full saturation with water/humidity (physical saturation based on electrolysis temperature/pressure conditions). E.g. up to 4 Vol%, normally up to 2 Vol% of hydrogen, limited by safety shut-off limits (explosive range begins at about 4 Vol% $H_2$ in $O_2$ at 1 atm) and water in the same order of magnitude. The exact content of hydrogen and water humidity in the oxygen gas produced by water electrolysis largely depends on temperature and pressure operating conditions.

The presence of hydrogen and water/steam prevent the skilled person from using electrolysis oxygen in the process for preparing acetylene and/or synthesis gas by partial oxidation of hydrocarbons. Hydrogen has a much lower heating value than the hydrocarbons such as methane employed in the partial oxidation synthesis process so that lower acetylene yields due to lower flame temperature are expected. The same effect is expected from water due to the high heat capacity leading to an overall very negative impact when using electrolysis oxygen in acetylene production.

However, it was found by the inventors of the present invention that the presence of hydrogen in the oxygen employed in the process preparing acetylene and/or synthesis gas by partial oxidation of hydrocarbons does not negatively influence the process, but - surprisingly - has a positive effect on the soot formation as well as on the yield of acetylene.

The present invention therefore relates to a process for preparing acetylene and/or synthesis gas by partial oxidation of hydrocarbons with an oxidizing agent, comprising the steps:

> i) Mixing the hydrocarbons and the oxidizing agent, whereby a mixture of the hydrocarbons and the oxidizing agent is obtained;
> ii) Igniting the mixture of the hydrocarbons and the oxidizing agent in a burner, whereby the partial oxidation of the hydrocarbons takes place and a first cracking gas stream comprising acetylene is obtained;
> iii) Cooling the first cracking gas stream, whereby a second cracking gas stream comprising acetylene is obtained; and
> iv) Extracting the acetylene from the second cracking gas stream, wherein acetylene and/or synthesis gas are obtained;

wherein the oxidizing agent comprises $O_2$ and $H_2$, preferably at least 1 ppmv of hydrogen, based on the total volume of the oxidizing agent.

The inventors therefore found a new oxidizing agent for the preparation of acetylene and/or synthesis gas by partial oxidation of hydrocarbons comprising - in addition to oxygen - hydrogen, independent from the source of the oxygen.

Generally, acetylene and synthesis gas are obtained by the inventive process, preferably, the inventive process is a process for preparing acetylene and synthesis gas.

In the meaning of the present invention oxygen means $O_2$ and hydrogen means $H_2$.

However, the process of the present invention is particularly environmentally friendly in case that the oxidizing agent is obtained at least in part by water splitting, preferably by electrolysis. More preferably, the oxidizing agent is completely obtained from water splitting by electrolysis.

The water splitting, preferably the electrolysis, preferably uses energy generated at least in part from non-fossil resources. More preferably, the water splitting, preferably the water electrolysis uses energy generated completely from non-fossil resources.

The present invention further relates to a cracking gas stream obtainable by steps i), ii) and iii) of the process according to the present invention; and to a cracking gas stream comprising 5 to 20 Vol% (based on the dry cracking gas stream / without water) of acetylene and synthesis gas comprising hydrogen, CO, $CO_2$ and $CH_4$. The cracking gas stream is the second cracking gas stream mentioned in the inventive process.

The present invention further relates to a cracking gas stream comprising 5 to 15 Vol% (based on the dry cracking gas stream / without water) of acetylene in the cracking gas stream. Furthermore the invention relates to synthesis gas comprising $H_2$, CO, $CO_2$ and $CH_4$, having a total cradle to gate product carbon footprint (PCF(mass-allocated) of acetylene and synthesis gas) of < 1.1 kg CO2e/kg acetylene and synthesis gas stream, preferably < 1.0 kg $CO_2$e/kg acetylene and synthesis gas stream, more preferably < 0.9 kg $CO_2$e/kg acetylene and synthesis gas stream.

Further, the present invention relates to acetylene obtainable by the process according to the present invention and to acetylene having a total cradle to gate product carbon footprint PCF (mass-allocated) of acetylene of < 1.1 kg $CO_2$e/kg acetylene, preferably < 1.0 kg $CO_2$e/kg acetylene, more preferably < 0.9 kg $CO_2$e/kg acetylene.

Due to the mass allocation, the numerical value PCF of acetylene, synthesis gas and acetylene+synthesis gas (cracking gas stream) is identical (unit = mass-specific: kg $CO_2$e/kg product).

The present invention further relates to a synthesis gas obtainable by the process according to according to the present invention and to a synthesis gas comprising $H_2$, CO, $CO_2$ and $CH_4$, wherein the separated synthesis gas stream has a $\delta^{80}O$ value of < 22 ‰, preferably, < 20 ‰, more preferably < 18 ‰, referred to the international standard VSMOW (Vienna- Standard-Mean-Ocean- Water), preferably obtained according to the process of the present invention.

The separated synthesis gas stream according to the present invention generally comprises (based on the dry cracking gas stream / without water) >50 Vol% $H_2$, >20 Vol% CO, <7 Vol% $CO_2$, <6 Vol% $CH_4$ and <3 Vol% of other gases (various hydrocarbon crack products and inert gases such as nitrogen, argon etc.) wherein the sum of $H_2$, CO, $CO_2$, $CH_4$ and other gases is 100Vol%.

The present invention further relates to the use of an oxidizing agent comprising $O_2$ and $H_2$, preferably obtained at least in part by water splitting, preferably by electrolysis, the water splitting, preferably the electrolysis, preferably using energy generated at least in part from non-fossil resources for the preparation of acetylene and synthesis gas.

The present invention further relates to the use of the inventive acetylene or the acetylene obtained by the inventive process for the preparation of of butynediol, butanediol, butenediol, polybutylene terephthalate (PBT), polybutylene adipate terephthalate (PBAT), tetrahydrofurane (THF), polytetrahydrofurane (polyTHF), polyester-based thermoplastic polyurethanes (TPUs), polyether-based TPUs, gamma-butyrolactone, pyrrolidine, vinylpyrrolidone, polyvinylpyrrolidone, N-methylpyrrolidone, vinyl ether, polyvinyl ether, terpenes and downstream products thereof, and in welding processes and cutting of metals.

The present invention further relates to the use of the inventive synthesis gas and the synthesis gas obtained according to the process of the present invention for the preparation of methanol, fuel applications, formaldehyde, acetic acid, olefins, sodium methylate, methylation products, dimethylterephthalate, methylamine, methyl mercaptane, polyoxymethylene, butynediol, methylendiphenyldiisocyanate, neopentylglycol, phenol formaldehyde resins, urea-condensation resins, melamine resins, acetone resins, chloroacetic acid, acetic acid ester, methylisipropylketone, acetic acid anhydride, dimethylsulfide, methanesulfonic acid and downstream products.

The present invention further relates to a process for preparing 1,4-butanediol comprising the following steps:

(a) reacting acetylene with formaldehyde in the presence of a catalyst, whereby a reaction mixture containing 2-butyne-1,4-diol is obtained;
(b) hydrogenating the 2-butyne-1,4-diol, whereby a reaction mixture containing 1,4-butanediol is obtained; and
(c) optionally isolating 1,4-butanediol from the reaction mixture, preferably by distillation. wherein the acetylene in step (a) is at least in part the inventive acetylene or the acetylene obtained by the process according to the present invention.

The present invention therefore further relates to a process for preparing formaldehyde from a synthesis gas comprising:

oxidation of the methanol obtained in the inventive process mentioned above to formaldehyde with oxygen, air or a mixture thereof in the presence of a catalyst;
and to a process for preparing formaldehyde from a synthesis gas comprising:

(A) conversion of said synthesis gas on a copper-containing catalyst to methanol, where the synthesis gas is at least in part the inventive synthesis gas or the synthesis gas obtained by the process of the present invention;
(B) oxidation of the methanol to formaldehyde with oxygen, air or a mixture thereof in the presence of a catalyst.

According to the Greenhouse Gas Protocol (WBCSD, WRI, 2011) greenhouse gas emissions are categorized into so called scope 1, scope 2 and scope 3 parts.

Scope 1 are direct $CO_2e$ emissions resulting from production processes in the plants that are owned or controlled by the reporting company. They arise from e.g.,

• emissions from chemical reactions,
• emissions stemming from waste treatment w/o energy use (e.g., flares),
• emissions from fuel and residues incineration in company-owned process plants.

Scope 2 are indirect $CO_2e$ emissions resulting from the generation of purchased energy such as electricity, steam, heat and cooling available via e.g. grid-connections and used as utilities by the company's plants.

Scope 3 upstream $CO_2e$ emissions are the sum of all indirect emissions resulting e.g., from the use of purchased raw materials from other suppliers including indirect emissions from transport up to the cradle of generation and extraction of fuels consumed by all product-processing plants in the complete value chain. It is important to note, that this can encompass the emissions of much more than one company since the total emissions of e.g. purchased raw materials up to the cradle must be included. This sum of indirect emissions are also called backpack emissions.

Scope 3 downstream $CO_2e$ emissions include the indirect greenhouse gas emissions within the company's value chain related to sold goods and services and emitted after they leave the company's ownership or control. This includes e.g. additional transportation and transformation related emissions up to the final disposal waste-related emissions at the final product's end-of-life. However, in the present application the "material carbon" in the polymer product is considered as the only add-on $CO_2$ emissions for the scope 3 downstream (end of life "grave") emissions; all other e.g. transportation and use-related emissions in the products customer and consumer use phase are out of scope.

The product carbon footprint (PCF) sums up the total greenhouse gas (GHG) emissions generated by a product over the different stages of its life cycle. For example, a cradle-to-gate (partial) PCF considers all the processes from extraction of resources through manufacturing of precursors and the making of the final product itself up to the point where it leaves the company gate (all product related direct GHG emissions, including removals, from Scopes 1, 2 and 3 upstream). A cradle-to-grave PCF covers the complete life cycle of the product, including the emissions from the use phase and end-of-life of the product (all product related direct GHG emissions, including removals, from Scopes 1, 2 and 3 upstream and downstream).

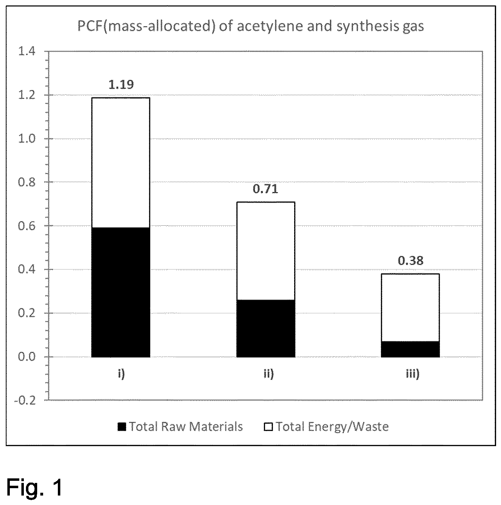

In Fig. 1 the system boundary definition according to the GHG Protocol are shown.

Unless otherwise indicated, PCF in this application refers to cradle-to-gate PCF. By "total cradle-to-gate PCF" is meant the total PCF of the corresponding acetylene and/or synthesis gas from cradle to gate.

By using the input and output ratios based on the total value product stream (definitions see Example 1 below) the resulting PCF values mentioned in the application for acetylene and synthesis gas are defined as mass-allocated PCF for the total value product mix acetylene+synthesis gas (AOG2)(dry) for the co-production of acetylene and synthesis gas via partial combustion of hydrocarbons with oxygen.

By using the input and output ratios based on the total value product stream (definitions see above and below) the resulting PCF values as follows are defined as mass-allocated PCF for the total value product mix acetylene+AOG2(dry) for the co-production of acetylene and synthesis gas via partial combustion of hydrocarbons with an oxidizing agent. This mass-allocated PCF is then identical to the PCF of both products acetylene and synthesis gas (same numbers for both products) because it is a "specific figure" specified in the unit kg CO2e/kg product.

One important factor for reducing the global greenhouse gas emission, especially the $CO_2$ emission is a quantitative knowledge about how much greenhouse gas emissions are associated with a product along the production value chain and along its life cycle. A Product Carbon Footprint (PCF) can help to find answers.

The Product Carbon Footprint (PCF) is the most established method for determining the climate impact of a product and a number of methods for calculating the PCF are known in the art. US2022/0107618 A1, US2022/0108327 A1 and US2022/0108326 A1 for example relate to computer-implemented methods for determining the carbon footprint of a product in production processes in a production plant, in particular of a product in interconnected production processes.

The Product Carbon Footprint helps to identify, analyze and, with the right measures, reduce or (ideally) completely avoid the climate-relevant impacts arising in the form of greenhouse gas emissions.

The relevant PCFs of acetylene and synthesis gas were calculated based on the following Guideline: "The Product Carbon Footprint Guideline for the Chemical Industry" launched by TfS (Together for Sustainability), Version 2.0, November 2022 (https://www.tfs-initiative.com/) (in the following: "TfS guideline"). The Guideline is "Audit-ready", ISO-compliant, and accepted by the GHG Protocol and further "Open source", useful for global industries using chemical materials.

As mentioned above, the PCF values mentioned in the application for acetylene and synthesis gas are defined as mass-allocated PCF for the total value product mix acetylene+synthesis gas (AOG2)(dry) for the co-production of acetylene and synthesis gas via partial combustion of hydrocarbons with oxygen.

The unique Verbund system of the applicant creates efficient value chains that extend from basic chemicals all the way to consumer products. This makes it possible to gather reliable data about greenhouse gas (GHG) emissions generated by a product in and beyond the company-owned Verbund, especially generated by acetylene and synthesis gas obtained via partial combustion of hydrocarbons with oxygen and upstream compounds in its preparation process, over the different stages of its life cycle, from cradle-to-gate (scopes 1, 2 and 3 upstream).

The Verbund system (Verbund configuration) is described as a site configuration that networks production facilities, energy flow, logistics, and infrastructure to run an overall process with lower energy consumption and higher yields and thus also generally lower PCF products due to avoided emissions compared to stand-alone production facilities.

As mentioned above, there are different routes to produce acetylene. A lot of energy is always needed because acetylene is a high-calorific molecule, i.e. during its preparation energy must be added to the system. This is according to the present invention done by partial oxidation process (BASF; developed by Sachsse and Bartholomé), which is for example described in the documents mentioned above. In case of methane as feedstock in the partial oxidation about 60% of the methane is partially oxidized by oxygen to carbon monoxide and hydrogen. This partial oxidation in an oxygen-enriched flame provides the energy to prepare acetylene generally at temperatures of around 1500-1600°C.

The process for preparing acetylene and/or synthesis gas by partial oxidation of hydrocarbons according to the present invention is generally carried out in a reactor system comprising a mixing unit, a burner block (also called "burner"), a combustion chamber and a quenching device. Such devices are described, for example, in Ullmann's Encyclopedia of Industrial Chemistry, 2012 Wiley-VCH Verlag GmbH & Co. KGaA, Weinheim, "Acetylene", 4.2.1, DE 875198, DE 1051845, DE 1057094, DE 4422815 and WO 2015/028539.

The mixer/burner block (also called "burner")/combustion chamber/quench combinations generally used for the inventive process - hereinafter referred to simply as reactor or acetylene reactor when reference is made to the combination - are explained in the documents mentioned above.

Step i)

Step i) comprises mixing the hydrocarbons and the oxidizing agent, whereby a mixture of the hydrocarbons and the oxidizing agent is obtained.

Suitable hydrocarbons are any hydrocarbons which are sufficiently volatile. The hydrocarbons may contain only one type of hydrocarbons but may be as well mixtures of hydrocarbons. Preferred hydrocarbons are selected from the group consisting of natural gas, methane, ethane, propane, butane, LPG (Liquefied Petroleum Gas), naphtha and mixtures thereof. More preferred hydrocarbons are selected from the group consisting of natural gas, methane, LPG (Liquefied Petroleum Gas), naphtha and mixtures thereof. Most preferred are natural gas, generally comprising predominantly methane.

Fossil-based conventional hydrocarbons can be used as well as synthetic methane prepared by "Power-to-X" processes with $CO_2$ and hydrogen as main raw materials. Preferably synthetic methane respectively the employed hydrogen is prepared using at least partially renewable energy and the $CO_2$ raw material is preferably captured from industrial flue gases (Carbon Capturing) or air (e.g. Direct Air Capturing, DAC). Mixtures of fossil-based and synthetic hydrocarbons are preferred as well.

In the case that natural gas is employed as hydrocarbon, every natural gas composition may be employed. Suitable natural gas contains for example at least 85 volume percent methane. The remaining components of such natural gas are usually ethane, propane, iso-butane, n-butane, n-pentane, iso-pentane and n-hexane.

Further, the hydrocarbons may contain small amounts of other gases besides the hydrocarbons, especially nitrogen, carbon dioxide, carbon monoxide, water, hydrogen sulfide as well as noble gases such as helium and/or argon.

The oxidizing agent according to the present invention comprises $O_2$ and $H_2$, preferably at least 1 ppmv of $H_2$, more preferably 1.5 to 20,000 ppmv of $H_2$, most preferably 2 to 15,000 ppmv of $H_2$ based on the total volume of the oxidizing agent.

The oxidizing agent according to the present invention may comprise 0 to <0.5 Vol% H2O, preferably 0.1 to <1 Vol% H2O, more preferably 1 to <50000 ppmv H2O, further more preferably 2.5 to <2500 ppmv H2O, most preferably 5 to <1000 ppmv H2O based on the total volume of the oxidizing agent.

The oxidizing agent according to the present invention may comprise 0 to <1500 ppmv N2, preferably 0.1 to <1000 ppmv N2, more preferably 0.5 to <500 ppmv N2 based on the total volume of the oxidizing agent. Most preferably, no N2 is present in the oxidizing agent.

It has been found by the inventors of the present invention that the presence of hydrogen in the oxygen employed in the process preparing acetylene and/or synthesis gas by partial oxidation of hydrocarbons does not negatively influence the process, but - surprisingly - has a positive effect on the soot formation as well as on the yield of acetylene.

The molar ratio of oxygen in the oxidizing agent and the hydrocarbons may be adjusted according to the desired acetylene to soot weight ratio and the highest possible acetylene yield under the given plant specific boundary conditions such as plant off-gas and plant waste water treatment design and local environmental discharge limits. If a higher soot generation is permissible, the oxygen feed may be reduced at constant hydrocarbon feed usually leading to increased acetylene yields depending on the plant configuration and process conditions.

Generally, at an oxygen ratio $\lambda$ 0.31, the process is conducted such that the concentration of acetylene in the dry cracking gas is at an optimum (e.g. >8 Vol%). At the same time, the soot loading of the cracking gas is also at a maximum. At an oxygen ratio $\lambda$>0.31 the process is optimized for lower soot production, however, yield of acetylene is reduced.

According to the present application, oxygen ratio $\lambda$ is understood to mean the ratio of the molar amount of oxygen actually present to the stoichiometrically necessary molar amount of oxygen required for the full combustion of the feedstocks.

According to the present invention, feedstocks are all combustible feedstocks, thus the hydrocarbons as well as the hydrogen present in the oxidizing agent.

Preferably, the inventive process is carried out at an oxygen ratio of $\lambda$>0.25 to $\lambda$<0.34, more preferably of $\lambda$>0.28 to $\lambda$<0.33. By said oxygen ratio a very high acetylene to soot weight ratio and a small soot production is achieved.

Preferably the feed ratio of the oxidizing agent to hydrocarbon feeds to the acetylene synthesis reactor are controlled as such, that the oxygen ratio is constant, preferably, the control is (semiautomatic. For example, the hydrocarbon feed (e.g. natural gas) can be used as reference value and "oxygen ratio control" of the oxidizing agent feed can be applied.

The following feed flow control concept for the continuous acetylene production process can be applied: Oxidizing agent feed (e.g. flow rate of electrolysis oxygen in $Nm^3$/h or kg/h) equipped with an online/inline hydrogen gas analysis sensor (e.g. concentration of H2 in Vol%) and automated flow control using the hydrocarbon feed (e.g. flow rate of natural gas in $Nm^3$/h or kg/h) as reference value and oxidizing agent feed running with ratio-control keeping the "oxygen ratio" constant, for example to setpoint $\lambda$= 0.3230. "Oxygen ratio" means the ratio of the molar amount of oxygen actually present to the stoichiometrically necessary amount of oxygen required for the full combustion of the feedstocks, whereby feedstocks in this respect are hydrocarbons and hydrogen.

The oxygen ratio $\lambda$ can be calculated "online" in the automated control system of the production line by implementing the appropriate calculation formula as stipulated above. In order to hold $\lambda$ constant over time, the controller is automatically adjusting the oxidizing agent flow rate, thereby compensating possible fluctuations in the gas compositions and flow rates and explicitly considering a variable H2 concentration as combustible feedstock on top of the hydrocarbons. Additional online/inline measurement instrumentation may be implemented as well if needed, e.g. measurement of gas feed densities and/or gas composition analytics of the hydrocarbon feed.

Alternatively, also the oxidizing agent feed can be set as the reference value feed, however using the hydrocarbon feed is preferred. Instead of using $\lambda$ directly, also other calculative ratios can be used in practice for example fitting to available instrumentation and output signals or simplified and proprietary ratio control strategies resulting in the same flow ratio control as by using the oxygen ratio λ.

Such an automated oxygen ratio control can be implemented for example in the distributed control system (DCS) of the acetylene production plant. The oxygen ratio λ is defined above. The concentration of hydrogen in the oxidizing agent feed is measured for example with online/inline analytics instrumentation allowing time-resolved online-calculation of the exact oxygen ratio. In case of changing hydrocarbon gas compositions this feed may also be equipped with online concentration instrumentation. Instead of online analytics, alternatively in case of less frequent fluctuations in the feed compositions, periodic offline analytics may also be used for one or both feeds in order to correct the flow ratio from time to time. The inventors clearly found that running acetylene production at constant oxygen ratio, as defined above, enables running at constantly optimal acetylene yield with simultaneously low soot production even with varying oxidizing agent and hydrocarbon feed compositions. This automated control strategy enables an optimal total acetylene yield and minimizes emissions over longer periods of time.

The mixing of the hydrocarbons and the oxidizing agent in step i) is carried out in the mixing unit of the acetylene reactor, generally ensuring intimate homogeneous mixing.

The hydrocarbons and the oxidizing agent are generally pressurized and added to the mixing unit in form of feedstock streams. The feedstock streams - one stream comprising the hydrocarbons and the other stream comprising the oxidizing agent - are generally first preheated. Preferably, the hydrocarbons and the oxidizing agent are preheated separately before the mixing in step i). The preheating temperature is dependent upon the hydrocarbon used. Typically, it is heated up to the order of magnitude of the auto-ignition temperature of the hydrocarbon. The preheating temperature for both feedstock streams is generally 150°C to 700°C, preferably 200°C to 650°C. In the case of use of natural gas, the preheating temperature is typically in a range of from about 500°C to 650°C. In the case of use of naphtha, the preheating temperature is typically in a range of from about 300°C to 500°C.

The oxidizing agent employed in the process of the present invention is preferably obtained at least in part by water splitting, preferably by electrolysis. The water splitting, preferably the electrolysis, preferably uses energy generated at least in part from non-fossil resources.

The term "at least in part from non-fossil resources" means that part of the energy can still be produced from fossil fuels, preferably from natural gas, since combustion of natural gas causes much lower carbon dioxide emission per megajoule of energy produced than combustion of coal. However, the share of energy produced from fossil fuels should be as low as possible, preferably ≤ 50%, more preferably ≤ 30%, most preferably ≤ 20%, further most preferably ≤ 10% of the energy in the water splitting, preferably electrolysis is generated from fossil resources. Further most preferably, the energy in the water splitting, preferably electrolysis is generated exclusively from non-fossil resources.

Also, the energy required in step i)\*, iii) and/or iv), preferably in step i)\* and in one, two or all further of steps iii) and iv), more preferably in steps i)\*, iii) and iv) is generated at least in part from non-fossil resources. However, also in step i)\*, iii) and iv) the portion of energy produced respectively provided from fossil fuels should be as low as possible, preferably ≤ 50%, more preferably ≤ 30%, most preferably ≤ 20%, further most preferably ≤ 10% of the energy in step i)\*, iii) and iv) is generated from fossil resources.

\*including the separate pressurization as well as preheating of the hydrocarbons and the oxidizing agent before the mixing in step i)

Step ii) is not mentioned in the paragraphs above and below, since step ii) requires no energy at all, since it is an exothermic step. The ignition generally happens "automatically" thanks to feeding an ignitable mixture.

Further preferably, at least 50%, preferably at least 70%, more preferably at least 80%, further more preferably at least 90% and most preferably 100% of the total required energy input used in step i)\*, iii) and/or iv), preferably in step i)\* and in one, two or all further of steps iii) and iv), more preferably in all steps i)\*, iii) and iv) is generated from non-fossil resources.

\*including the separate pressurization as well as preheating of the hydrocarbons and the oxidizing agent before the mixing in step i)

It should be considered, however, that the hydrocarbons employed as raw material in step i) are generally produced from fossil resources - in the embodiments described above and below. However, it is also possible to employ hydrocarbons in step i) generated at least in part from non-fossil resources, wherein the term "at least in part from non-fossil resources" has the meaning that at least 50%, preferably at least 70%, more preferably at least 80%, further more preferably at least 90% and most preferably 100% of the total required hydrocarbons in step i) of the process of the present invention is generated from non-fossil resources.

Most preferably, in the inventive process for preparing acetylene and/or synthesis gas the energy in step i)\*, iii) and/or iv), preferably in step i)\* and in one, two or all further of steps iii) and iv), more preferably in all steps i)\*, iii) and iv) is generated exclusively from non-fossil resources.

\*including the separate pressurization as well as preheating of the hydrocarbons and the oxidizing agent before the mixing in step i)

In a further preferred embodiment, at least 50%, preferably at least 70%, more preferably at least 80%, further more preferably at least 90% and most preferably 100% of the total required energy input used in the process of the present invention is generated from non-fossil resources.

Generally, the energy used in steps i)*, iii) and iv) and the energy in the water splitting, preferably electrolysis is used in form of heating energy and/or electrical power.

*including the separate pressurization as well as preheating of the hydrocarbons and the oxidizing agent before the mixing in step i)

Preferably, the energy is generated at least in part from non-fossil resources selected from solar energy (thermal, photovoltaic and concentrated solar power), wind power, hydroelectricity (tidal power, wave power, hydroelectric dams, In-river-hydrokinetics), geothermal energy, heat captured by heat pumps, bioenergy (biofuel, biomass), the renewable part of waste energy sources, nuclear energy and mixtures thereof.

In a further embodiment, the energy is generated at least in part from non-fossil resources selected from renewable resources, preferably selected from solar energy (thermal, photovoltaic and concentrated solar power), wind power, hydroelectricity (tidal power, wave power, hydroelectric dams, In-river-hydrokinetics), geothermal energy, heat captured by heat pumps, bioenergy (biofuel, biomass), the renewable part of waste and mixtures thereof.

The types of energy resources mentioned above are generally known by a person skilled in the art.

In one preferred embodiment of the inventive process, the energy from non-fossil resources used in the process according to the invention is generated at least in part by nuclear energy. The nuclear energy is generally obtained by fission.

Fission occurs when a neutron enters a larger atomic nucleus, forcing it to excite and spilt into two smaller atoms- also known as fission products. Additional neutrons are also released that can initiate a chain reaction. When each atom splits, a tremendous amount of energy is released. Uranium and plutonium isotopes are most commonly used for fission reactions in nuclear power reactors because they are easy to initiate and control. The energy released by fission in these reactors heats water into steam. The steam is used to spin a turbine to produce carbon-free electricity.

In one preferred embodiment of the inventive process, the energy used in the process according to the invention is generated at least in part from wind power. Wind power can be used to run wind turbines. Modern utility-scale wind turbines range from around 600 kW to 9 MW of rated power. The power available from the wind is a function of the cube of the wind speed, so as wind speed increases, power output increases up to the maximum output for the particular turbine. Areas where winds are stronger and more constant, such as offshore and high-altitude sites, are preferred locations for wind farms.

In one further preferred embodiment of the inventive process, the energy used in the process according to the invention is generated at least in part from solar power, particularly preferred from photovoltaic systems. A photovoltaic system converts light into electrical direct current (DC) by taking advantage of the photoelectric effect. Concentrated solar power (CSP) systems use lenses or mirrors and tracking systems to focus a large area of sunlight into a small beam. CSP-Stirling currently has the highest efficiency among all solar energy technologies.

In one preferred embodiment of the inventive process, the energy used in the process according to the invention is generated at least in part from hydropower. There are many forms of hydropower. Traditionally, hydroelectric power comes from constructing large hydroelectric dams and reservoirs. Small hydro systems are hydroelectric power installations that typically produce up to 50 MW of power. They are often used on small rivers or as a low-impact development on larger rivers. Run-of-the-river hydroelectricity plants derive energy from rivers without the creation of a large reservoir. The water is typically conveyed along the side of the river valley (using channels, pipes and/or tunnels) until it is high above the valley floor, whereupon it can be allowed to fall through a penstock to drive a turbine.

Wave power, which captures the energy of ocean surface waves, and tidal power, converting the energy of tides, are two forms of hydropower with future potential.

In one further preferred embodiment of the inventive process, the energy used in according to the invention is generated at least in part from geothermal energy. Geothermal energy is the heat that comes from the sub-surface of the earth. It is contained in the rocks and fluids beneath the earth's crust and can be found as far down to the earth's hot molten rock, magma.

To produce power from geothermal energy, wells are dug a mile deep into underground reservoirs to access the steam and hot water there, which can then be used to drive turbines connected to electricity generators. There are three types of geothermal power plants; dry steam, flash and binary.

Dry steam is the oldest form of geothermal technology and takes steam out of the ground and uses it to directly drive a turbine. Flash plants use high-pressure hot water into cool, low-pressure water whilst binary plants pass hot water through a secondary liquid with a lower boiling point, which turns to vapor to drive the turbine.

In one further preferred embodiment of the inventive process, the energy used in the process according to the invention is generated at least in part from biomass. Biomass is biological material derived from living, or recently living organisms. It most often refers to plants or plant-derived materials which are specifically called lignocellulosic biomass. As an energy source, biomass can either be used directly via combustion to produce heat (e.g. heat from fermentation processes) or electricity, or indirectly after converting it to various forms of biofuel and gas. Conversion of biomass to biofuel can be achieved by different methods which are broadly classified into: thermal, chemical, and biochemical methods. Wood was the largest biomass energy source as of 2012; examples include forest residues - such as dead trees, branches and tree stumps -, yard clippings, wood chips and even municipal solid waste. Industrial biomass can be grown from numerous types of plants, including miscanthus, switchgrass, hemp, corn, poplar, willow, sorghum, sugarcane, bamboo, and a variety of tree species, ranging from eucalyptus to oil palm (palm oil).

Plant energy is produced by crops specifically grown for use as fuel that offer high biomass output per hectare with low input energy. The grain can be used for liquid transportation fuels while the straw can be burned to produce heat or electricity. Biomass can be converted to other usable forms of energy such as methane gas or transportation fuels such as ethanol and biodiesel. Rotting garbage, and agricultural and human waste, all release methane gas - also called landfill gas or biogas. Crops, such as corn and sugarcane, can be fermented to produce the transportation fuel, ethanol. Biodiesel, another transportation fuel, can be produced from left-over food products such as vegetable oils and animal fats.

Biopower technologies convert renewable biomass fuels into heat and electricity using processes like those used with fossil fuels. There are three ways to harvest the energy stored in biomass to produce biopower: burning, bacterial decay, and conversion to a gas or liquid fuel. Biopower can offset the need for carbon fuels burned in power plants, thus lowering the carbon intensity of electricity generation. Unlike some forms of intermittent renewable energy, biopower can increase the flexibility of electricity generation and enhance the reliability of the electric grid.

Further preferably, at least part of the energy used in the process of the present invention is utilized from excess energy generated in other exothermic chemical processes than the process steps of the present invention (typical examples: acrylic acid, formaldehyde or ethylene oxide production), for example in a chemical "Verbund" production setup distributing the excess energy e.g. as steam to endothermic process consumers. Excess steam can substitute steam generated by fossil fuels and be allocated as $CO_2e$ credits to the exothermic process source thereby closing the $CO_2$-balance as described in the "TfS PCF guideline". Such a Verbund plant is preferably supplied with additional energy generated from non-fossil resources.

Various methods for certification and tracking of the "energy source mix" have been set up based on local legislations. Certificates such as "Non-Fossil Certificate Contracts" are common practice for tracking the ratio of non-fossil energy used in industrial processes and related products (https://www.ekoenergy.org/ecolabel/criteria/tracking/).

Preparation (generation) of oxygen:

The oxygen used in the oxidizing agent may generally be obtained by any process known in the art. Generally, the oxygen may be obtained by separation from the atmosphere. Water ($H_2O$) can also be split into hydrogen ($H_2$) and oxygen ($O_2$) using electrolysis or solar energy. Said processes are generally known in the art.

It is also possible that the oxygen obtained by separation from the atmosphere is obtained at least in part or exclusively by energy generated from non-fossil resources.

The oxygen obtained by separation from the atmosphere is free of hydrogen. For providing the oxidizing agent used in the present application, it is therefore necessary to add hydrogen to said oxygen separated from the atmosphere.

However, the oxygen obtained by water splitting, preferably by electrolysis, comprises a small amount of hydrogen.

Therefore, oxidizing agent employed in the process of the present invention is preferably obtained at least in part by water splitting, preferably by electrolysis. Mixtures with conventional oxygen from air separation can be applied as per supply/demand availability. More preferably, the total amount of the oxidizing agent employed in the process of the present invention is obtained by water splitting, preferably by electrolysis.

The water splitting, preferably the electrolysis, preferably uses energy generated at least in part from non-fossil resources. More preferably, the water splitting, preferably the electrolysis, exclusively uses energy generated from non-fossil resources.

The term "at least in part from non-fossil resources" is explained above.

The term "at least in part by water splitting" means that part of the oxygen can still be produced by other processes, generally by separation from the atmosphere. However, the portion of oxygen in the oxidizing agent in step i) produced by other methods than by water splitting should be as low as possible.

Preferably, in step i) of the process of the present invention $\leq$ 50%, preferably $\leq$ 30%, most preferably $\leq$ 20%, further most preferably $\leq$ 10% of the oxygen in the oxidizing agent is produced by other methods than by water splitting. In one embodiment, the oxygen in the oxidizing agent in step i) is produced exclusively by water splitting, preferably by electrolysis.

The oxygen in the oxidizing agent used in step i) in the process of the present invention, which is obtained by water splitting, preferably by electrolysis, but using energy generated from fossil resources, may generally obtained by using any energy generated from fossil resources known in the art. A preferred fossil resource is natural gas, since combustion of natural gas causes much lower carbon dioxide emission per megajoule of energy produced than combustion of for example coal. However, the portion of energy produced from fossil fuels should be as low as possible in the process of the present invention. Most preferably, in the case that the oxygen in the oxidizing agent in step i) is obtained by water splitting, preferably by electrolysis, the energy is fully generated from non-fossil resources.

Water splitting using energy based on renewable sources is an environmentally friendly method for production of oxygen because it uses renewable $H_2O$ and the oxygen is a surplus by-product, which is generally released into the environment, in the production of renewable hydrogen ("green hydrogen"). The electrolysis of 9 tons of water gives 1 ton of hydrogen and 8 tons of oxygen gas, but usually only the hydrogen is used as a value product. There are no further significant amounts of by-products in the water splitting.

A recent review article gives a detailed overview of the currently most relevant technologies focusing on commercial large-scale implementation of water electrolysis for hydrogen production in the context of global energy transformation (H. Ozcan et al, Recent advances, challenges, and prospects of electrochemical water-splitting technologies for net-zero transition, Cleaner Chem. Eng. 8 (2023) 100115 - https://doi.org/10.1016/j.clce.2023.100115).

The water splitting can generally performed by known processes like electrolysis; photocatalytic water splitting, also called photoelectrochemical (PEC) water splitting; chemically assisted electrolysis, e.g. carbon/hydrocarbon assisted water electrolysis (CAWE); radiolysis; ultrasound; thermolysis, especially via solar energy, e.g involving using solar concentrators to directly collect solar energy to heat water; pyrolysis on biomass; nuclear-assisted thermolysis, e.g in a high-temperature gas-cooled reactor (HTGR); thermochemical cycle combining solely heat sources (thermo) with chemical reactions to split water into its hydrogen and oxygen components, e.g the sulfur-iodine cycle (S-I cycle); ferrosilicon method; photobiological water splitting and mixtures thereof.

Generally, any water source can be used in the water splitting.

Preferably, the water splitting is performed by electrolysis and/or photocatalytic water splitting, more preferably by electrolysis.

In photocatalytic (photoelectrochemical (PEC)) oxygen (and hydrogen) is produced from water using sunlight and one or more photocatalysts, in general specialized semiconductors called photoelectrochemical materials, which use light energy to directly dissociate water molecules into hydrogen and oxygen.

The photocatalysts (semiconductor materials) used in the photocatalytic (PEC) process are similar to those used in photovoltaic solar electricity generation, but for photocatalytic (PEC) applications the photocatalyst (semiconductor) is generally immersed in a water-based electrolyte, where sunlight energizes the water-splitting process.

PEC reactors can for example be constructed in panel form (similar to photovoltaic panels) as electrode systems or as slurry-based particle systems.

The most preferred water electrolysis generally utilizes as electrical power direct current (DC) at least in part from non-fossil energy resources.

One suitable water electrolysis process is alkaline water electrolysis. Oxygen (and hydrogen) production by alkaline water electrolysis is a well-established technology up to the megawatt range for a commercial level. In alkaline water electrolysis initially at the cathode side two water molecules of alkaline solution (KOH/NaOH) are reduced to one molecule of hydrogen ($H_2$) and two hydroxyl ions (OH-). The produced $H_2$ emanates from the cathode surface in gaseous form and the hydroxyl ions (OH$^-$) migrate under the influence of the electrical field between anode and cathode through the porous diaphragm to the anode, where they are discharged to half a molecule of oxygen ($O_2$) and one molecule of water ($H_2O$). Alkaline electrolysis operates at lower temperatures such as 30-100°C with alkaline aqueous solution (KOH/NaOH) as the electrolyte, the concentration of the electrolyte being about 20% to 40 %. The diaphragm in the center of the electrolysis cell separates the cathode and anode and also separates the produced gases from their respective electrodes, avoiding the mixing of the produced gases.

The operating temperature of the alkaline water electrolysis is typically around 80 °C and systems of up to 750 $Nm^3$ $H_2$/h are available. These systems use an aqueous solution of potassium hydroxide (KOH) as an electrolyte with a typical concentration of 20-40% and achieve current densities of 0.25-0.45 A/cm$^2$. Rectangular and circular electrodes and cells with an active area of up to approximately 3 m$^2$ have also been used. Also pressurized electrolyzers are known (e.g. manufactured by Lurgi GmbH (Air Liquide Global E&C Solutions Germany GmbH)) that supply hydrogen and oxygen below 30 bar. Such pressurized electrolyzer produces 760 Nm$^3$/h of hydrogen, which corresponds to an electrical output of approximately 3.6 MW. Lifetimes of up to 90,000 h are achieved by the stack, with the electrodes and diaphragms needing to be replaced after this period.

An overview of alkaline water electrolysis powered by renewable energy is given in J. Brauns and T. Turek in Processes, 8(2) (2020), pp. 248.

In one further embodiment of the inventive process, oxygen (and hydrogen) is provided by polymer electrolyte membrane water electrolysis. Variants of polymer electrolyte membrane water electrolysis are proton exchange membrane water electrolysis (PEMWE, PEM water electrolysis) and anion exchange membrane water electrolysis (AEMWE, AEM water electrolysis).

Low-temperature electrolysis processes based on alkaline and PEM technologies can be used to establish large, powerful systems (1-100 MW). In contrast to PEM electrolysis, a number of alkaline electrolysis designs have been used for several decades at various scales. However, polymer electrolyte membrane water electrolysis like PEM and AEM do have advantages over the alkaline electrolysis.

According to the NOW study by the Fraunhofer Institute for Solar Energy Systems (ISE, Frei-burg), the voltage efficiency of the stack amounts to 62-82% in relation to the higher heating value (HHV). At less than 20%, the lower partial load operation is especially critical for its flexible application in combination with renewable energy sources. This is a result of the diaphragms that are used, which facilitate the mixture of hydrogen and oxygen due to diffusion, which in turn leads to safety-related switch offs. Another disadvantage of alkaline electrolyzers as compared with PEM electrolysis is the costly gas treatment of the product gases, for which expensive noble metals must be used.

In contrast to alkaline water electrolysis, PEM electrolysis with proton-conducting membranes uses platinum group metals for the electrodes. Due to the use of dense membranes as an electrolyte and the possibility of integration with recombination catalysts, the systems can be operated at 0-100% power; however, in technical facilities the lower threshold is limited to approximately 5% of nominal power due to the internal consumption of peripheral components.

All large PEM electrolysis manufacturers are working on the development of MW systems with various stack concepts. For instance, a 1 MW PEM electrolyzer with a single stack and a nominal power of 1 MW is known (Hydrogenics). For the most part, the commercially-available stacks only operate with current densities < 2.0 A/cm$^2$. In these systems, approximately 6 mg/cm$^2$ of iridium or ruthenium is required for the anode, and approximately 2 mg/cm$^2$ of platinum is required for the cathode. In contrast to alkaline electrolysis, the lifetime of PEM electrolysis stacks is estimated at <20,000 h. However, PEM electrolysis stacks with a lifetime of more than 50,000 h are known (Proton Onsite; HOGEN C series).

The PEM water electrolyzer utilizes a solid polymer electrolyte (SPE) to conduct protons from the anode to the cathode while insulating the electrodes electrically. Under standard conditions the enthalpy required for the formation of water is 285.9 kJ/mol. One portion of the required energy for a sustained electrolysis reaction is supplied by thermal energy and the remainder is supplied through electrical energy.

The half reaction taking place on the anode side of a PEM water electrolyzer is commonly referred to as the Oxygen Evolution Reaction (OER). Here the liquid water reactant is supplied to a catalyst where it is oxidized to oxygen, protons and electrons.

The half reaction taking place on the cathode side of a PEM water electrolyzer is commonly referred to as the Hydrogen Evolution Reaction (HER). Here the protons that have moved through the membrane are reduced to gaseous hydrogen.

PEMs can be made from either pure polymer membranes or from composite membranes, where other materials are embedded in a polymer matrix. One of the most common and commercially available PEM materials is the fluoropolymer PFSA (e.g. Nafion®, a DuPont product). While Nafion® is an ionomer with a perfluorinated backbone like Teflon, there are many other structural motifs used to make ionomers for proton-exchange membranes. Many use polyaromatic polymers, while others use partially fluorinated polymers.

An overview over PEM water electrolysis is given in S. Kumar and V. Himabindu, Material Science for Energy Technologies 2 (2019), pp. 4442 - 4454.

The AEM water electrolysis technology adopts low-cost catalytic materials, as in alkaline electrolysis, and a solid polymer electrolyte architecture, as in PEM electrolysis technology. AEM electrolysis technology operates in an alkaline environment (pH ~ 10), making it possible the use modest non-noble-metal electrocatalysts (i.e. platin group metal free catalysts = PGM free catalysts), whilst accommodating a zero-gap architecture. The membrane used in this type of electrolysis is a polymeric membrane, containing quaternary ammonium salts. It is relatively inexpensive and has low interaction with atmospheric $CO_2$.

Catalysts:

As examples for hydrogen evolution reaction (HER) catalysts, catalysts based on Ni-Mo alloyed materials are suitable.

As examples for oxygen evolution reaction (OER) catalysts, high activity of transition metal mixed oxides are suitable. Specific examples are CuxCo3_xO4, NiCo2O4:Fe and Ni-Fe alloys on Ni foam supports, for example the PGM-free catalysts (Ni-Fe, Ni-Mo, Ni/(CeO2-La2O3)/C and CuxCo3_xO4).

Membranes and ionomers:

The chemical stability of AEMs under alkaline conditions has improved markedly due to the development of stabilized functional groups on the polymer backbone. This allows the use of such membranes in AEM electrolysis at higher temperatures for long periods. Suitable membranes and ionomers are known by a person skilled in the art and for example described in the review mentioned below. One example is the commercial membrane Tokuyama A201.

Membrane electrode assembly preparation and cell performance:

The physical and electrochemical characterization of the membrane electrode assembly prepared by either the catalyst-coated substrate or the catalyst-coated membrane (CCM) method suggests that the CCM is preferable because improvements in ionic conductivity far outweigh any improvements in electronic conductivity.

Liquid electrolyte: Pure water feeds generally result in poor current densities while 1% $K_2CO_3$ or dilute KOH solutions give good results. A good electrolysis performance is achieved with a 1% $K_2CO_3$ electrolyte. It is therefore preferable that the water electrolyte comprises 0.1 to 2 wt% $K_2CO_3$ or KOH.

An overview over anion exchange membrane water electrolysis is given in H. A. Miller et al., Sustainable Energy Fuels, 2020, 4, pp. 2114 - 2133.

Beside the alkaline water electrolysis, the AEM and PEM, a further commercially available electrolysis technology is the solid oxide electrolysis (SOE).

SOEC (solid oxide electrolysis cell) feeds water into the cathode and the water undergoes water reduction reaction (WRR), which converts water into hydrogen gas and oxide ions. This hydrogen gas is later brought to purification modules to separate hydrogen gas from the remaining water. Then, the oxide ions migrate from cathode to anode and they release electrons to external circuit to become oxygen gas *via* oxygen evolution reaction (OER). Typically, the operating temperatures for SOFCs are from 800 to 1,000 °C, because high temperatures are required to thermally activate the migration of oxide ions and to facilitate electrochemical reactions on both electrodes. As a result, the overall efficiency is improved. The SOEC is for example described in K. Kamlungsua et al., FUEL CELLS 20, 2020, No. 6, 644-649.

Preferably, the electrolysis for obtaining oxygen (and hydrogen) is a water electrolysis, more preferably PEM water electrolysis, alkaline water electrolysis, or AEM water electrolysis.

In a further preferred embodiment, the electrolysis for obtaining oxygen (and hydrogen) is a solid oxide water electrolysis (SOE).

Step ii)

The mixture of the hydrocarbons and the oxidizing agent obtained in step i) is brought to reaction, generally after passing through a diffuser which is preferably present in the burner block (also called "burner").

Step ii) of the inventive process therefore comprises igniting the mixture of the hydrocarbons and the oxidizing agent in a burner, whereby the partial oxidation of the hydrocarbons takes place and a first cracking gas stream comprising acetylene is obtained.

The "igniting" mentioned above is generally a "self-igniting" at the temperature present in the burner.

In the process according to the invention, the required thermal energy is generally provided directly by partial oxidation of the hydrocarbon mixture (the primary fuel).

Generally the mixture of the (generally preheated) hydrocarbons and the (generally preheated) oxidizing agent obtained in step i) has a temperature at which self-ignition occurs.

The flame temperature is generally depending on the hydrocarbon and oxidizing agent compositions used as feed as well as the oxygen ratio $\lambda$ and various process conditions such as preheating temperatures, pressure and flow rates / throughput.

The flame reaction temperature in step ii) is generally 1100 to 1900 °C, preferably 1200 to 1800 °C, more preferably 1400 to 1700 °C.

Suitable burner for the acetylene synthesis are known in the art (see for example Ullmann's Encyclopedia of Industrial Chemistry, 2012 Wiley-VCH Verlag GmbH & Co. KGaA, Weinheim, "Acetylene", 4.2.1, DE 875198, DE 1051845, DE 1057094, DE 4422815 and WO 2015/028539).

As an example, each burner has its own set of preheaters, and six to nine burners feed one purification train. The gases are mixed in the burner's mixing chamber or diffuser, giving a specific oxygen ratio $\lambda$ as mentioned above and then enter a flow stabilizing grid. Both the exothermic oxidation of some of the hydrocarbons and the pyrolysis of the hydrocarbons to give acetylene take place in the flame.

The grid, or burner block, generally comprises a multitude of small diameter tubes through which the mixed gases flow; water flowing around the tubes provides cooling. Ignition of the gases occurs at the outlets of the burner block tubes to form a flame that fills the space below. Immediately below the flame, generally water spray quenches the hot gas to preserve the acetylene formed in the flame (see step iii)).

Generally, the conversion of oxygen in the flame is complete, and >90%, preferably >93%, most preferably >95%, further most preferably >96 to 99.5% of the hydrocarbons react.

The reaction time in step ii) generally varies depending on flow rates of hydrocarbon mixture and oxidizing agent, reaction temperature and pressure, and is generally from 0.0001 to 0.05 seconds, preferably from 0.001 to 0.01 seconds. This reaction time, or induction time, varies depending on the feedstock being used, with feeds with higher methane concentrations having shorted reaction times as they are more reactive.

The pressure in the burner is generally slightly above atmospheric.

Acetylene is a metastable molecule, so if it resides at such high temperatures for too long a time, it will continue to react quantitatively to produce soot. The cracked gas stream obtained in step ii) (first cracking gas stream) must consequently be cooled down after a sufficient, but not excessive residence time. The residence time is the reaction time mentioned above.

Step iii)

Step iii) comprises: Cooling the first cracking gas stream, whereby a second cracking gas stream comprising acetylene is obtained.

Generally, the cooling of the first cracking gas stream may be carried out by any method known in the art (see for example in Ullmann's Encyclopedia of Industrial Chemistry, 2012 Wiley-VCH Verlag GmbH & Co. KGaA, Weinheim, "Acetylene", 4.2).

Preferably, the cooling in step iii) is carried out by quenching with water.

The gas (cracked gas) from the burner is generally cooled to a temperature below 100°C, preferably to about 80 °C to 100 °C. The gas quench can be designed in a single step or in multiple steps in order to optimize heat recovery and acetylene yield.

Generally, the water-saturated burner gas obtained in step ii) (first cracking gas stream comprising acetylene) is cooled by direct contact with water, e.g. in a cooling column.

The water quench process is known in the art (see for example DGMK-Tagungsbericht 2013-2, ISBN 978-3-941721-32-6, M. Vicari, "BASF and Acetylene - 70 Years of Reppe Chemistry - Long-Standing Reliability and Promising Future - and now, the only Natural Gas Based Clean Technology for Acetylene Production", p. 99-102).

A water-quench acetylene plant generally has several parts, the preheaters for both hydrocarbon and oxygen feedstocks, the cracked gas generation (burner), the water-quench section and subsequent compression separation sections for acetylene and synthesis gas recovery. Soot formed in the burner is captured in the quench-water and removed in cooling columns and subsequent offgas and quench-water-circuit cleaning plant sections. For a general plant configuration overview see Fig. 11 and Fig. 13 in Ullmann's Encyclopedia of Industrial Chemistry, 2012 Wiley-VCH Verlag GmbH & Co. KGaA, Weinheim, "Acetylene".

In the generation section the cracked gas is produced, generally containing mainly acetylene, carbon monoxide, hydrogen, residual hydrocarbon, e.g. methane, and minor amounts of higher acetylenes and aromatics. Some of the soot formed in the reaction and water is separated here too.

The next step is the compression generally up to around 10 bars. This step also removes the rest of the soot from the gas stream, in a closed water-quench process (see below).

The open water-quench process generally needs electrofilters to remove the soot from the cracked gas stream and soot decanters to separate the soot from the process water.

The separation section of the plant follows next, where the acetylene is separated from the synthesis gas (also called acetylene off-gas, AOG) and a higher acetylenes stream.

The synthesis gas stream contains all the carbon monoxide, hydrogen, carbon dioxide, methane, ethane, ethylene and further inerts (e.g. N2, Ar, He).

Typical components in the higher acetylenes stream are components like di-acetylene, vinyl-acetylene and propyne including residual acetylene itself.

The water quench may be a closed water-quench or an open water-quench. Preferably, the quenching with water is a closed water-quench process. A suitable closed water-quench process is for example described in US 5,824,834.

As mentioned above, the open water-quench process generally needs electrofilters to remove the soot from the cracked gas stream and soot decanters to separate the soot from the process water. The soot decanters and the open cooling tower are the source of the serious organic components emissions of the open water-quench process.

With the process of the present invention, low amounts of soot are built at a high acetylene yield. Due to this, in the closed water-quench process the electrofilters and the soot decanters can be omitted and the cooling water system can be operated in a closed loop.

In the closed water-quench process, the remaining soot in the cooled cracked gas stream generally after the cooling columns is generally separated for example by electrofilters or other gas filter means. The soot in the quench water loop is generally separated by flocculation and filtration or any other means of solid/liquid separation.

The stream obtained in step iii) is the second cracking gas stream according to the present invention.

The second cracking gas stream preferably comprises 5 to 20 Vol% (based on the dry cracking gas stream / without water) of acetylene and synthesis gas comprising hydrogen CO, $CO_2$ and residual $CH_4$, residual oxygen and small amounts of higher acetylenes components (see above) as well as inert gases such as N2, Ar and He.

The present invention further relates to a cracking gas stream obtainable by steps i), ii) and iii) of the process according to the present invention.

The present invention further relates to a cracking gas stream comprising 5 to 20 Vol% (based on the dry cracking gas stream / without water) of acetylene and synthesis gas comprising hydrogen, CO, $CO_2$ and $CH_4$, having a total cradle to gate product carbon footprint (PCF(mass-allocated) of acetylene and synthesis gas) of < 1.1 kg CO2e/kg acetylene and synthesis gas stream, preferably < 1.0 kg $CO_2$e/kg acetylene and synthesis gas stream, more preferably < 0.9 kg $CO_2$e/kg acetylene and synthesis gas stream.

Step iv)

Step iv) comprises extracting the acetylene from the second cracking gas stream, wherein acetylene and/or synthesis gas are obtained.

Generally, the acetylene extraction from the second cracking gas stream is known in the art and for example described in DGMK-Tagungsbericht 2013-2, ISBN 978-3-941721-32-6, M. Vicari, "BASF and Acetylene - 70 Years of Reppe Chemistry - Long-Standing Reliability and Promising Future - and now, the only Natural Gas Based Clean Technology for Acetylene Production", p. 99-102. Suitable apparatus for the acetylene extraction are also known by a person skilled in the art and mentioned for example in the document mentioned before as well as in Ullmann's Encyclopedia of Industrial Chemistry, 2012 Wiley-VCH Verlag GmbH & Co. KGaA, Weinheim, "Acetylene", 4.2. The extraction is for example carried out in one or more columns.

Generally, the acetylene is separated from the second cracking gas stream (comprising acetylene and synthesis gas) and higher acetylenes after compression, preferably by selective absorption into a solvent after compression so that the partial pressure of acetylene is generally kept below 1.4 bar to ensure safe processing out of explosion risk domains.

After adsorption into the solvent, acetylene is generally recovered by depressurizing the liquid and subsequent stripping, usually at elevated temperatures. The solvent is generally recycled in the acetylene separation process.

An example for an extraction step iv) is mentioned in the following:

In a first column, the pre-scrubber, mainly diacetylene, higher acetylenes and aromatics are removed by a low amount of NMP (n-methyl-pyrrolidone). In a subsequent main scrubber a larger amount of NMP is used to capture acetylene entirely in the liquid while crude synthesis gas is exiting at the top of the column. From the obtained solution acetylene is stripped off, generally in a multi-step stripper/degassing process. Then the acetylene-containing NMP from the main scrubber is generally flashed to a stripper. A first flash gas is generally carbon dioxide and small amounts of acetylene. This top product of the stripper is recycled to the cracked gas (the second cracking gas stream) before the compressors. Therefore, the carbon dioxide goes to the synthesis gas stream (acetylene off-gas), the top product of the main absorber.

The acetylene is desorbed from the NMP and generally withdrawn in a side stream of the stripper. Generally, the obtained acetylene is scrubbed with water to recover NMP solvent before going to a product gas storage holder. Further purification to increase the acetylene purity for example to about 99.7 volume percent can for example be accomplished by scrubbing with sulfuric acid and sodium hydroxide solutions.

Instead of NMP, other solvents or solvent mixtures miscible with water can be used in step iv). According to the present invention, the acetylene is therefore extracted from the second cracking gas stream with NMP, DMF (dimethylformamide), ammonia, kerosene, octane, methanol, acetone or mixtures thereof.

As mentioned above, by the process of the present invention, acetylene having a distinctively low PCF (total cradle to gate product carbon footprint PCF (mass-allocated)) is obtained. The terms "mass allocated" and "product carbon footprint are explained above and below.

The present invention therefore relates to acetylene having a total cradle to gate product carbon footprint (mass-allocated) of acetylene of < 1.1 kg $CO_2$e/kg acetylene, preferably < 1.0 kg $CO_2$e/kg acetylene, more preferably < 0.9 kg $CO_2$e/kg acetylene.

Due to the mass allocation, the numerical value PCF of acetylene, synthesis gas and acetylene+synthesis gas (cracking gas stream) is identical (unit = mass-specific: kg $CO_2$e/kg product).

The synthesis gas obtained in the inventive process (AOG) is a value product which is generally used for other purposes. Examples are feedstocks for the synthesis of chemical intermediates, such as methanol, OXO aldehydes, etc. as well as power generation. Examples for uses of synthesis gases are known in the art and for example given in https://en. wikipedia.org/wiki/Syngas and below.

Also, the synthesis gas is specific, due to the specific inventive process.

Therefore, the present invention further relates to a synthesis gas obtainable by the process according to according to the present invention.

The separated synthesis gas stream according to the present invention generally comprises (based on the dry cracking gas stream / without water) >50 Vol% $H_2$, >20 Vol% CO, <7 Vol% $CO_2$, <6 Vol% $CH_4$ and <3 Vol% of other gases (various hydrocarbon crack products and inert gases such as nitrogen, argon etc. and residual oxygen) wherein the sum of $H_2$, CO, $CO_2$, $CH_4$ and other gases is 100 Vol%.

As mentioned above, due to the mass allocation, the numerical value PCF of acetylene, synthesis gas and acetylene+synthesis gas (cracking gas stream) is identical (unit = mass-specific: kg $CO_2$e/kg product).

The present invention therefore further relates to synthesis gas having a total cradle to gate product carbon footprint (mass-allocated) of synthesis gas of < 1.1 kg $CO_2$e/kg synthesis gas, preferably < 1.0 kg $CO_2$e/kg synthesis gas, more preferably < 0.9 kg $CO_2$e/kg synthesis gas.

In a preferred embodiment of the inventive process, the oxidizing agent is obtained at least in part by water splitting, preferably by electrolysis. As mentioned above, oxygen is produced as a by-product of water electrolysis, which is usually released into the environment without further use.

In physical organic chemistry, a kinetic isotope effect is the change in the reaction rate of a chemical reaction when one of the atoms in the reactants is replaced by one of its isotopes. Formally, it is the ratio of rate constants $k_L / k_H$ for the reactions involving the light ($k_L$) and the heavy ($k_H$) isotopically substituted reactants (isotopologues). This change in reaction rate is a quantum mechanical effect that primarily results from heavier isotopologues having lower vibrational frequencies compared to their lighter counterparts. In most cases, this implies a greater energetic input needed for heavier isotopologues to reach the transition state, and consequently, a slower reaction rate.

Isotopic rate changes are most pronounced when the relative mass change is greatest, since the effect is related to vibrational frequencies of the affected bonds. For instance, changing a hydrogen atom (H) to its isotope deuterium (D) represents a 100 % increase in mass. However, isotope effects also exist for $^{18}O/^{16}O$.

It is observed that by the electrolysis of water, the $^{18}O$ atom content of the evolved oxygen gas is lower than the $^{18}O$ atom content in the oxygen obtained by separation from the atmosphere.

The reason therefore is the kinetic isotope effect mentioned above, which is relevant in the present invention for two effects.

First, in the electrolysis of water, the heavy oxygen $^{18}O$ content of the obtained oxygen gas is lower than in the water to be electrolyzed.

Second, whereas the starting material of the oxygen obtained by separation from the atmosphere is ambient air, the starting material of the oxygen preferably used in the present invention is water ("water electrolysis"). However, the isotopic composition of atmospheric oxygen ($^{18}O/^{16}O_2$) in air is enriched relative to (oceanic) water (Dole effect). Therefore, the heavy oxygen $^{18}O$ content of the oxygen obtained by water electrolysis is also for this reason lower than that of the oxygen obtained by separation from the atmosphere.

The low heavy oxygen $^{18}O$ content of the electrolysis oxygen has also an impact on the downstream products, i.e. on the synthesis gas obtained in the inventive process as well as on the downstream products of said synthesis gas.

The present invention therefore relates to a synthesis gas comprising hydrogen, CO, $CO_2$ and $CH_4$, wherein the synthesis gas stream has a $\delta^{80}O$ value of < 22 ‰, preferably, < 20 ‰, more preferably < 18 ‰, referred to the international standard VSMOW (Vienna- Standard- Mean-Ocean- Water), preferably obtained by the process according to the present invention.

The present invention therefore further relates to the use of the $\delta^{80}O$ value in oxygen and downstream compounds based on oxygen for tracing the origin of preparation of oxygen and downstream compounds based on oxygen, wherein the compounds are preferably synthesis gas and downstream products based on synthesis gas, like methanol, fuel applications, formaldehyde, acetic acid, olefins, sodium methylate, methylation products, dimethylterephthalate, methylamine, methyl mercaptane, polyoxymethylene, butynediol, methylendiphenyldiisocyanate, neopentylglycol, phenol formaldehyde resins, urea-condensation resins, melamine resins, acetone resins, chloroacetic acid, acetic acid ester, methylisipropylketone, acetic acid anhydride, dimethylsulfide, methanesulfonic acid and downstream products.

A process for tracing the origin of preparation of oxygen and downstream compounds based on oxygen by determining the $\delta^{80}O$ value in oxygen and downstream compounds based on oxygen, wherein the compounds are preferably synthesis gas and downstream products based on synthesis gas, like methanol, fuel applications, formaldehyde, acetic acid, olefins, sodium methylate, methylation products, dimethylterephthalate, methylamine, methyl mercaptane, polyoxymethylene, butynediol, methylendiphenyldiisocyanate, neopentylglycol, phenol formaldehyde resins, urea-condensation resins, melamine resins, acetone resins, chloroacetic acid, acetic acid ester, methylisipropylketone, acetic acid anhydride, dimethylsulfide, methanesulfonic acid and downstream products.

In - generally known - oxygen-18 isotope ($^{18}O$) analysis, the absolute content is usually not determined, in contrast to the determination of chemical constituents.

In the case of stable isotopes, an isotope abundance ratio ($^{18}O/^{16}O$) is measured and compared with an international standard.

The deviation of the isotope abundance ratio of the sample compared to that of the standard is given as $\delta$ value in per thousand (‰). $\delta^{80}O$ values refer to the international standard VSMOW (Vienna- Standard- Mean-Ocean- Water).

As the majority of natural waters contain fewer "heavy" isotopes than the reference, the $\delta$ values are often negative.

For the precise determination of the stable oxygen-18 only small sample quantities (a few ml) are required. The analytical measurement is carried out in a mass spectrometer (see the general method mentioned below).

Vienna Standard Mean Ocean Water (VSMOW) is an isotopic water standard defined in 1968 by the International Atomic Energy Agency. Despite the somewhat misleading phrase "ocean water", VSMOW refers to pure water ($H2O$) and does not include any salt or other substances usually found in seawater. VSMOW serves as a reference standard for comparing hydrogen and oxygen isotope ratios, mostly in water samples. Very pure, distilled VSMOW water is also used for making high accuracy measurement of water's physical properties and for defining laboratory standards since it is considered to be representative of "average ocean water", in effect representing the water content of Earth.

Generally, the synthesis gas obtained in the inventive process (AOG) can be further purified or directly used by downstream-processes known in the art when used as value product, especially when used as feedstock for the synthesis of chemical intermediates.

The acetylene according to the present invention or obtained by the process of the present invention is a high-value product useful in numerous applications. See for example Ullmann's Encyclopedia of industrial chemistry, 2012 Wiley-VCH Verlag GmbH & Co. KGaA, Weinheim, ""Acetylene", Chapter3.1. Industrially Important Reactions and 3.2. Other Reactions; Derivatives.

The present invention further relates to the use of an oxidizing agent comprising $O_2$ and $H_2$, preferably obtained at least in part by water splitting, preferably by electrolysis, the water splitting, preferably the electrolysis, preferably using energy generated at least in part from non-fossil resources for the preparation of acetylene and synthesis gas.

Acetylene is an attractive basic chemical, and it is used for the preparation of numerous chemical compounds as well as in welding and cutting processes.

The present invention therefore further relates to the use of the inventive acetylene or the acetylene obtained by the inventive process for the preparation of butynediol, butanediol, butenediol, polybutylene terephthalate (PBT), polybutylene adipate terephthalate (PBAT), tetrahydrofurane (THF), polytetrahydrofurane (polyTHF), polyester-based thermoplastic polyurethanes (TPUs), polyether-based TPUs, gamma-butyrolactone, pyrrolidine, vinylpyrrolidone, polyvinylpyrrolidone, N-methylpyrrolidone, vinyl ether, polyvinyl ether, terpenes and downstream products thereof; and in welding processes and cutting of metals.

The present invention further relates to butynediol, butanediol, butenediol, polybutylene terephthalate (PBT), polybutylene adipate terephthalate (PBAT), tetrahydrofurane (THF), polytetrahydrofurane (polyTHF), polyester-based thermoplastic polyurethanes (TPUs), polyether-based TPUs, gamma-butyrolactone (GBL), pyrrolidine, vinylpyrrolidone, polyvinylpyrrolidone, N-methylpyrrolidone, vinyl ether, polyvinyl ether, terpenes and downstream products thereof obtained from the acetylene according to the present invention or obtained from the acetylene obtained by the process according to the present invention.

One important chemical which can be obtained from acetylene is butanediol, especially 1,4-butanediol (BDO or 1,4-BDO).

1,4-BDO is an important raw material for example in manufacturing spandex, plastics, elastic fibers, and films. Besides its use as a commodity chemical, 1,4-BDO can be used as a starting chemical for other derivatives like THF, polyTHF, polyesters like PBT and PBAT, polyester-based TPUs, polyether-based TPUs, and GBL. In high-temperature condition, THF is synthesized from 1,4-BDO in the presence of phosphoric acid, while GBL is formulated by the dehydrogenation of 1,4-BDO in the presence of soluble ruthenium catalyst. Processes for preparing THF, polyTHF, polyesters like PBT and PBAT, polyester-based TPUs, polyether-based TPUs, and GBL from 1,4-BDO are known in the art.

Also, processes for the preparation of 1,4-BDO from acetylene are known in the art.

For the industrial synthesis of 1,4-BDO, acetylene is in a first step converted to 2-butyne-1,4-diol with formaldehyde solution (e.g. 10-30 wt% formaldehyde in water) on a catalyst, preferably a copper catalyst, e.g. a bismuth-modified, silica-supported copper(I) acetylide catalyst. The reaction temperature is generally from 50 to 100°C, preferably from 65 to 90 °C. The reaction pressure is generally from 0.5 to 15 bar, preferably from 0.9 to 10 bar. Generally, propargyl alcohol is formed as one by-product, which is separated by distillation and returned to the reaction stage. The selectivity to 2-butyne-1,4-diol is generally >85%, preferably >90 % in relation to formaldehyde. The first step generally takes place in a cascade of two or more fixed-bed reactors, which are generally operated in the sump or trickle mode.

In a second reaction step, the resulting 2-butyne-1,4-diol is hydrogenated, generally in stages, to 1,4-butanediol. The hydrogenation generally takes place at temperatures of 50 to 250°C, preferably 70 to 220°C The pressures are generally 100 to 350 bar, preferably 150 to 300 bar (fixed-bed or trickle-phase hydrogenation) or 5 to 80 bar, preferably 10 to 65 bar (suspension or liquid-phase hydrogenation). The hydrogenation is generally carried out in the presence of a catalyst, e.g. Raney nickel catalysts, preferably containing additional promoters such as copper or chromium. The selectivity to 1,4-butanediol is generally > 90%, preferably ≥ 95 % relative to 2-butyne-1,4-diol. The hydrogenation is preferably carried out in jet nozzle, stirred tank or bubble column reactors. The obtained product (1,4-BDO) is purified and worked up as known in the art, e.g. by multi-stage distillation in rectification columns.

In the processes mentioned above, the inventive acetylene or the acetylene obtained by the process according to the present invention is preferably employed.

The present invention therefore further relates to a process for preparing 1,4-butanediol comprising the following steps:

(a) reacting acetylene with formaldehyde in the presence of a catalyst, whereby a reaction mixture containing 2-butyne-1,4-diol is obtained;

(b) hydrogenating the 2-butyne-1,4-diol, whereby a reaction mixture containing 1,4-butanediol is obtained; and

(c) optionally isolating 1,4-butanediol from the reaction mixture, preferably by distillation. wherein the acetylene in step (a) is at least in part the inventive acetylene or the acetylene obtained by the process according to the present invention.

Preferably, the formaldehyde in step (a) is prepared at least in part from the inventive synthesis gas or the synthesis gas obtained by the process according to the present invention.

Suitable reaction conditions and catalysts employed in steps (a), (b) and (c) in the process for preparing 1,4-butanediol are known by a person skilled in the art and mentioned above.

A suitable process for preparing formaldehyde from the inventive synthesis gas or the synthesis gas obtained by the process of the present invention is mentioned below.

The 1,4-BDO obtained by the inventive process can be used for the preparation of downstream products, especially for the preparation of THF, polyTHF, polyesters like PBT and PBAT, polyester-based TPUs, polyether-based TPUs, and GBL.

Also, the inventive synthesis gas and the synthesis gas obtained according to the process of the present invention is a product of value.

The present invention further relates to the use of the inventive synthesis gas and the synthesis gas obtained according to the process of the present invention for the preparation of methanol, fuel applications, formaldehyde, acetic acid, olefins, sodium methylate, methylation products, dimethylterephthalate, methylamine, methyl mercaptane, polyoxymethylene, butynediol, methylendiphenyldiisocyanate, neopentylglycol, phenol formaldehyde resins, urea-condensation resins, melamine resins, acetone resins, chloroacetic acid, acetic acid ester, methylisipropylketone, acetic acid anhydride, dimethylsulfide, methanesulfonic acid and downstream products.

The present invention further relates to methanol, fuel applications, formaldehyde, acetic acid, olefins, sodium methylate, methylation products, dimethylterephthalate, methylamine, methyl mercaptane, polyoxymethylene, butynediol, methylendiphenyldiisocyanate, neopentylglycol, phenol formaldehyde resins, urea-condensation resins, melamine resins, acetone resins, chloroacetic acid, acetic acid ester, methylisipropylketone, acetic acid anhydride, dimethylsulfide, methanesulfonic acid and downstream products obtained from the inventive synthesis gas or obtained from the synthesis gas according to the present invention.

Generally, the synthesis gas according to the present invention and the downstream compounds based on said synthesis gas, especially methanol, fuel applications, formaldehyde, acetic acid, olefins, sodium methylate, methylation products, dimethylterephthalate, methylamine, methyl mercaptane, polyoxymethylene, butynediol, methylendiphenyldiisocyanate, neopentylglycol, phenol formaldehyde resins, urea-condensation resins, melamine resins, acetone resins, chloroacetic acid, acetic acid ester, methylisipropylketone, acetic acid anhydride, dimethylsulfide, methanesulfonic acid and downstream products, comprise a $\delta^{80}O$ value which is lower that the $\delta^{80}O$ value of said compounds based on synthesis gas obtained by conventional methods not based on electrolysis oxygen.

Two important chemicals which can be obtained from synthesis gas are methanol and formaldehyde.

Methanol is a chemical building block for numerous products, including plastics, paints, car parts and construction materials. Methanol also is a clean energy resource.

The processes for producing methanol from synthesis gas are classified according to the reaction pressure (high, medium and low pressure processes). Today, methanol is produced on an industrial scale from synthesis gas using low- or medium-pressure processes. Each process works with special catalysts and carbon monoxide/hydrogen ratios and under special reaction conditions. The exact reaction conditions and catalysts are known to the person skilled in the art.

The crude methanol produced in both the low-pressure and medium-pressure processes is generally partially contaminated with by-products. If the crude methanol is used for combustion in the energy sector, the purity of the crude methanol is generally sufficient. For further processing in the chemical industry, the raw methanol is generally processed, usually by distillation. Low-boiling components such as dimethyl ether are generally separated in a low-boiling column. The higher-boiling fractions are usually separated off as a bottom in a further distillation stage, generally in a high-boiling column, with methanol usually being drawn off overhead.

Suitable catalysts for the production of methanol from synthesis gas are known to those skilled in the art and are generally copper catalysts. Copper-zinc oxide-aluminum oxide catalysts are preferably used in both the low-pressure and medium-pressure processes, whereby the catalysts can be doped with cesium, for example. In the medium-pressure process, catalysts based on oxidic copper-chromium-zinc or chromium-zinc catalysts can also be used, for example.

The copper-zinc oxide-aluminum oxide catalysts are produced according to processes known to the skilled person, e.g. by co-precipitation starting from copper, zinc and aluminum hy-droxycarbonates. Further steps include washing, ageing, drying, calcination and activation by reduction in a hydrogen/nitrogen stream, whereby the copper oxide produced during calcination is reduced to the metal. Suitable processes are known to the skilled person. The production of catalysts based on oxidic copper-chromium-zinc or chromium-zinc catalysts is also known to the skilled person.

Low pressure process:

The low-pressure process is generally carried out on the basis of the above-mentioned copper-zinc oxide-aluminum oxide catalysts. Dimethyl ether, formic acid methyl ester and ethanol, which are generally distilled off, are generally produced in the ppm range as by-products of the reaction. The low-pressure process is generally carried out at a pressure of 20 to 120 bar. The temperature is generally 200 to 300 °C.

The reactor types used in the low-pressure process are, for example, fixed-bed tube bundle reactors in which the catalyst bed is located in tubes surrounded by boiling water. The tube diameter is generally designed in such a way that the temperature in the catalyst bed can be kept constant at around 5 to 10 °C. The resulting steam is used, for example, to heat the catalyst. The resulting steam is used, for example, to drive the compressors or to distil the raw methanol.

Medium pressure process:

The medium-pressure processes generally use both the above-mentioned copper-zinc oxide-alumina catalysts such as the low-pressure process and, for example, catalysts based on oxidic copper-chromium-zinc or chromium-zinc catalysts. A corresponding process works, for example, at a pressure of 100 to 250 bar. The temperature is generally 220 to 300 °C.

In the processes mentioned above, the inventive synthesis gas or the synthesis gas obtained by the process according to the present invention is preferably employed.

The present invention therefore further relates to a process for preparing methanol from a synthesis gas comprising:

conversion of said synthesis gas on a copper-containing catalyst, where the synthesis gas is at least in part the inventive synthesis gas or the synthesis gas obtained by the process of the present invention.

Suitable reaction conditions and catalysts employed in the process for preparing methanol are known by a person skilled in the art and mentioned above.

The methanol obtained by the inventive process can be used for example as clean energy as well as for the preparation of downstream products, especially for the production of formaldehyde, acetic acid, and methyl tert-butyl ether.

Formaldehyde is an important raw material for the chemical industry. Formaldehyde is used in the production of synthetic resins such as phenolic resins, urea resins and melamine resins, as well as in the production of polyols and polyoxymethylene. Formaldehyde is also used as a disinfectant and preservative and is used in numerous chemical synthesis processes. A large proportion of global methanol production is used for the synthesis of formaldehyde.

According to the present invention, the formaldehyde is produced starting from the methanol produced according to the process of the invention.

Formaldehyde ($CH_2O$) is produced on an industrial scale by the catalytic oxidation of methanol with oxygen, generally atmospheric oxygen (air).

Nowadays, two different large-scale processes are mainly used for this purpose: the formox process and the oxidative dehydrogenation of methanol. The processes are known in the art.

Formox process:

In the formox process, methanol is oxidized to form formaldehyde. In this process, methanol is generally mixed with an excess of atmospheric oxygen (air). The temperature is generally 250°C to 440°C. The preferred catalysts are iron oxide catalysts, molybdenum oxide catalysts or vanadium oxide catalysts. Preferably, oxides of molybdenum or iron are used in the catalyst at a molar ratio of 1.5 to 2.0 (Mo:Fe), wherein small amounts of oxides of vanadium, cobalt, phosphorus, chromium, and copper may also be included.

The reaction takes place for example in a fixed bed, vapor phase reactor.

In a typical process methanol, fresh air, and recycle gas are mixed together and vaporized for example in a evaporator/vaporizer. The ratio of the recycled gas to fresh air is generally approximately 2.5:1. Generally, recycle of the off-gas, which is rich in inerts, decreases the oxygen concentration and permits the use of relatively high methanol concentrations without creating an explosive mixture. Preferably, the oxygen concentration is kept at approximately 10 percent, while methanol is around six to nine percent, both on a molar basis.

The warm reactor feed gas is fed to the reactor, which generally has tubes filled with catalyst.

Methanol is oxidized over the metal oxide catalyst to form formaldehyde. Gas leaving the reactor generally consists largely of air and formaldehyde. In one design as an example, effluent gas is cooled from 250 °C to around 110 °C by indirect heat exchange in the evaporator before passing to the absorption column, where the formaldehyde is condensed and absorbed in the solution. The concentration of the solution can be varied from 37 to around 58 percent, depending upon the quantity of process water added and the tower design. Indirect cooling is supplied to the scrubbing plates and the process solution is recirculated through coolers at each stage of packing.

Oxidative dehydrogenation:

In the second production process, the oxidative dehydrogenation of methanol, silver catalysts are generally used. Two primary reactions occur during the conversion of methanol to formaldehyde (dehydrogenation and partial oxidation). The process is generally carried out at atmospheric pressure, generally with atmospheric oxygen. At temperatures of generally 600°C to 750°C, methanol is dehydrogenated to formaldehyde. During dehydrogenation, hydrogen atoms are split off from a chemical compound. The released hydrogen then reacts with atmospheric oxygen (air) to form water.

The present invention therefore further relates to a process for preparing formaldehyde from a synthesis gas comprising:

oxidation of the methanol obtained in the inventive process mentioned above to formaldehyde with oxygen, air or a mixture thereof in the presence of a catalyst.

The present invention further relates to a process for preparing formaldehyde from a synthesis gas comprising:

(A) conversion of said synthesis gas on a copper-containing catalyst to methanol, where the synthesis gas is at least in part the inventive synthesis gas or the synthesis gas obtained by the process of the present invention;

(B) oxidation of the methanol to formaldehyde with oxygen, air or a mixture thereof in the presence of a catalyst.

Suitable reaction conditions and catalysts employed in the process for preparing formaldehyde are known by a person skilled in the art and mentioned above.

The formaldehyde obtained by the inventive process can be used for example in the production of urea formaldehyde (UF) resins, phenol formaldehyde (PF) resins, melamine formaldehyde (MF) resins, polyoxymethylenes (POM), methylene dephenyl diisocyanate (MDI), 1,4-butanediol (BDO), pentaerythritol (Penta), hexamine, and paraformaldehyde (PFA).

The formaldehyde obtained by the inventive process can be used in the inventive process for preparing 1,4-butanediol.

The invention is further illustrated by the following examples.

Examples

The invention is further illustrated by the following examples.

Example 1: Determination of the total cradle-to-gate product carbon footprint (PCF) of acetylene and synthesis gas

Process for preparing acetylene and/or synthesis gas by partial oxidation of hydrocarbons with an oxidizing agent, comprising the steps:

i) Mixing the hydrocarbons and the oxidizing agent, whereby a mixture of the hydrocarbons and the oxidizing agent is obtained;

ii) Igniting the mixture of the hydrocarbons and the oxidizing agent in a burner, whereby the partial oxidation of the hydrocarbons takes place and a first cracking gas stream comprising acetylene is obtained;

iii) Cooling the first cracking gas stream, whereby a second cracking gas stream comprising acetylene is obtained; and

iv) Extracting the acetylene from the second cracking gas stream, wherein acetylene and/or synthesis gas are obtained;

wherein the oxidizing agent comprises $O_2$ and $H_2$, preferably at least 1 ppmv of $H_2$, based on the total volume of the oxidizing agent.

The total cradle-to-gate PCF data were determined based on the TfS Guideline* as follows:

* The Product Carbon Footprint Guideline for the Chemical Industry" launched by TfS (Together for Sustainability), Version 2.0, November 2022 (https://www.tfs-initiative.com/); relevant boundary conditions and definitions for the PCF calculations are documented as follows.

A) Compilation of input ratios per production step:

- Raw Materials, e.g. kg raw material/kg step product (for all major raw materials)

- Thermal Energy, e.g. kg steam/kg step product (total effective heating energy input for production step; if applicable, exothermal energy production is considered as negative input thus as steam credit in the respective production step as per TfS allocation rules)
- Electricity, e.g. kWh/kg step product (electrical power for production step)
- Waste Generation, e.g. waste material output in kg waste/kg step product and the effective carbon content in wt-%, for example as total organic carbon (TOC) in waste water (for all C-containing waste materials)

B) Compilation of backpack emissions for input raw materials and energy per production step:

- Raw Material PCF backpack, e.g. kg $CO_2$e/kg raw material (for all major raw materials)
- Thermal Energy backpack emissions, e.g. CO2e/kg steam
- Electricity backpack emissions, e.g. $CO_2$e/kWh
- Waste, considered with fixed factor $CO_2$/C molar mass ratio: 44/12 = 3.67 kg $CO_2$e/kg carbon waste

C) Calculation of the total cradle-to-gate product carbon footprint per step product:

- PCF Total Raw Materials = Sum of each Raw Material input ratio multiplied with its Raw Material PCF backpack (for all major raw materials of the step product)
- PCF Total Energy/Waste = Sum of each energy input ratio multiplied with its energy backpack emissions (for e.g. thermal and electrical energy used for the step product) and waste emissions are considered by multiplication of the waste material carbon output with the fixed $CO_2$/C factor explained above; for all C-containing waste materials)
- Total cradle-to-gate product carbon footprint = PCF Total Raw Materials + PCF Total Energy/Waste

Source of data used for the example calculations:

a) Acetylene and synthesis gas industrial production input ratios and total mass balance

- General overview by P. Pässler et al, Acetylene, in: Ullmann's Encyclopedia of Industrial Chemistry. Wiley-VCH Verlag, 2011, DOI: 10.1002/14356007.a01_097.pub4, chapter 4.2.1 "BASF Process (Sachsse-Bartholome), Table 6", idealized mass- and energy input ratios
- Scope: State-of-the art continuous partial combustion synthesis with closed water-quench mode and co-production of acetylene and synthesis gas (mainly CO and H2)
- Note: According to the current review by V. Galvita et al, Methane Conversion Routes - Status and Prospects, Green Chemistry Series No 76, Royal Society of Chemistry, 2024, chapter 4 "Conversion of Methane to Acetylene" this BASF process is "the most energy-efficient route to directly convert methane to acetylene to date". The Hüls arc process is only next best industrial alternative because it is "extremely energy intensive".
- Industrial scale operating point for production of ~25 t/d acetylene used for state-of-the art mass balance input ratios incl. process water and low soot-production (not shown in the above mentioned general overview of Ullmann's Encyclopedia): US 5,824,834 Example 1
- Auxiliary raw materials used e.g. for cracked gas and acetylene purification such as for example sulfuric acid, caustic NaOH and N-Methyl-Pyrrolidone (NMP) are only used in small quantities significantly <5% based on total material mass input. Therefore auxiliary raw materials are neglected in accordance with the TfS Guideline.
- Main raw material input is a hydrocarbon feed such as natural gas and an oxidizing agent in form of purified oxygen gas
- Product output of the Sachsse-Bartholome process is acetylene (C2H4) and synthesis gas (mainly CO and H2 as well as residual oxygen and H2O and CO2 from the partial oxidation reaction along with small amounts of residual CH4 and various other residual hydrocarbon crack products):
Simplified main reaction for methane:

$$CH4 + O2 \rightarrow C2H2 + CO + H2 \ (+ H2O + CO2 + Waste/Others)$$

- Produced CO2 and soot as quantified in US 5,824,834 Example 1 are considered as waste output
- In order to cover all additionally relevant remaining waste streams of the production process (especially coke and higher acetylenes gas) all the carbon emissions which are not shown in above references are disclosed in WO2013/186291, table page 8 "Emissionen offener Wasser-quench" and converted to total kg C/t acetylene; for these emissions different final waste-treatment processes can be used as per detail plant design and local environmental regulation requirements. Such a state-of-the-art plant configuration encompasses the closed water-quench mode for acetylene production which fulfills the current emission regulations especially for waste water (TOC) and offgas (VOC).

- The total acetylene process mass balance is closed by calculating the output of synthesis gas (here called AOG1, dry acetylene offgas) as follows:

AOG = Input_Hydrocarbon + Input_Oxidizing Agent + Output)Acetylene-Output_ProcessWater-Output_-Waste whereby Output_ProcessWater = water (H2O) from the partial oxidation reaction; usually measured via the process water mass balance, see US 5,824,834 Example 1.

$$Output\_Waste = Output\ soot + Output\ remaining\ waste\ (see\ above)$$

- For the synthesis gas product mix, here we define the AOG2 as relevant total dry value product acetylene offgas stream defined as follows:

$$AOG2 = AOG1 - ProcessCO2$$

whereby
ProcessCO2 = carbon dioxide (CO2) generated in the synthesis process by the partial oxidation reaction; measured e.g. via gas analysis in the acetylene offgas stream as shown as well in US 5,824,834 Example 1. In the present application, the term AOG2 (without CO2) is only introduced in order to allocate the produced CO2 (ProcessCO2) as waste to the pure value products acetylene and synthesis gas as per TfS requirements.
- Energy input for the acetylene synthesis process is considered from

a) Heating and flaring fuel, here from additional Natural Gas feed as input material given in GJ in Ullmann's Encyclopdia (additionally, the resulting CO2 emissions are considered as heating and flaring fuel waste)
b) Thermal energy, here steam as shown in Ullmann's Encyclopedia
c) Electric energy, here electricity as shown in Ullmann's Encyclopedia

All resulting input and output ratios based on the total value product stream (acetylene + AOG2) are shown in table 1

Table 1

| Input | Input ratio (kg RM or kg steam or kWh per kg TotalVP*) |
|---|---|
| Hydrocarbons for synthesis: Natural Gas (example composition: 98.04 Vol% CH4, 0.78 Vol% Ethane, 0.34 Vol% Propane, 0.8 Vol% N2, 0.04 Vol% CO2) | 0.676 |
| Oxidizing agent (High purity oxygen) | 0.847 |
| Heating and flaring fuel: Natural Gas | 0.054 |
| Thermal Energy (Steam) | 0.441 |
| Electricity (Electrical power) | 0.456 |
| | |
| **Output** | Output ratio (kg waste per kg TotalVP*) |
| Waste - Soot (as 100% carbon) | |
| Waste - Remaining waste (as 100% carbon) | 0.077 |
| Waste - CO2 production (as 100% carbon) | |
| Waste - Heating and flaring fuel CO2 emissions (as 100% carbon) | |
| *) RM = Raw Material; TotalVP = acetylene + AOG2 (TotalVP = total value product stream) | |

This data shows the total raw material and energy consumption ratios for state-of-the art continuous partial combustion acetylene and synthesis gas production technology with closed water-quench mode fulfilling currently highest environmental regulation standards. All input ratios imply ideal plant operations with constant nameplate capacity throughput without significant maintenance shutdown times, thus full plant utilization of a regular chemical commodity production plant. All practical deviations from these best-case conditions will result in higher input rations and thus higher PCF values.

4) Backpack emission data for acetylene and synthesis gas industrial production examples

**[0280]** - We consider acetylene production in Verbund with hydrocarbon supply via e.g. natural gas pipeline supply and on-site pure oxygen production connected to the acetylene plant via pipeline

| | Scenario i) Comparative | Scenario ii) Inventive | Scenario iii) Inventive |
|---|---|---|---|
| Hydrocarbons: Natural Gas for synthesis (as raw material) (example composition, see above) | assuming rough average 0.38 kg CO2e/kg NG; see a#), b#) and c#) for exemplary reference data and explanations of strong variability | same as scenario i) | assuming best-case 0.1 kg CO2e/kg NG; see a#), b#) and c#) |
| Oxidizing agent (High purity Oxygen) | d#) and also see e#) for reference data and explanations of strong variability; here assuming rough average 0.39 kg CO2e/kg oxidizing agent | assuming 50% use of oxygen from water splitting via electrolysis using solar and/or wind power as shown in #g): 50% of scenario i) = 0.20 kg CO2e/kg oxidizing agent | assuming 100% use of oxygen from water splitting via electrolysis using solar and/or wind power as shown in #g): 0 kg CO2e/kg oxidizing agent (Hydrogen and oxygen by-product coupled electrolytical production) |
| Heating and flaring fuel: Natural Gas | see above (Hydrocarbons: Natural Gas) | same as scenario i) | see above, scenario iii) |
| Thermal Energy (Steam) | Flannery et al.# WP22-16; "The Greenhouse Gas Index for Products in 39 Industrial Sectors"; Chapter 5.3; Table 1, Page 13; Fuel natural gas | assuming 50% of scenario i) due to partial use of thermal energy generated with non-fossil energy | assuming 10% of scenario i) due to predominant use of thermal energy generated with non-fossil energy |
| Electricity (Electrical power) | #f) US Energy Information Administration: U.S. electricity net generation and | assuming 50% of scenario i) due to increased use of electricity generated with non-fossil energy | Flannery et al.# WP22-16; "The Greenhouse Gas Index for Products in |
| | resulting CO2 emissions in 2021; Fuel natural gas; 0.44 kg CO2e/kWh | | 39 Industrial Sectors"; Chapter 5.3; Table 2, Page 13; assuming 0.03 CO2e/kWh due to use of advanced solar and wind based non-fossil energy mix |

Reference list:

Flannery et al.# = Brian P. Flannery, Jan W. Mares; Resources for the Future (independent, nonprofit research institution, Washington, DC): The Greenhouse Gas Index for Products in 39 Industrial Sectors, Sectoral Modules published September 2022; https://www.rff.org/publications/working-papers/the-greenhouse-gas-index-for-products-in-39-industrial-sectors/, downloaded February 2023.

#a) Website Ecocostvalue.com Stichting Sustainability Impact Metrics, partnership with Delft University of Technology, https://www.ecocostsvalue.com/EVR/img/Idemat2008+EI_V2-0.xls, table "full lists Ecoinvent" (file downloaded April 2023): Ecoinvent ecocosts of materials, products, energy, transport, prossessing, etc. calculated from ecoinvent v2):

- Natural gas: Various PCF carbon footprint values are published, for pipeline gas e.g. under "Materials, fuels, natural gas, gas by country, imports" or "Materials, fuels, natural gas, gas by country" - numbers are ranging from 0.05 to 0.8 (average 0.38) kg CO2e/kg NG, heavily depending on the source of gas and transportation distances, thus the data is extremely source supplier and destination country dependent; Note that the PCF of natural gas is also very sensitive to methane emissions from various losses during extraction/processing and pumping/pipelining with partially not completely conclusive or even unavailable data, also constantly in progress of being updated as emission/reporting standards are improving over time.
- Oxygen: "Oxygen, liquid, at plant/RER S", purity und technology as well as source of energy undisclosed, published at 0.407 kg CO2e/kg oxygen

#b) North Sea Transition Authority (NSTA), Natural gas carbon footprint analysis, fact sheet carbon footprint of UK natural gas imports (published 2023, nsta-gas-import-fact-sheet.pdf downloaded January 2024), https://www.nstauthority.co.uk/the-move-to-net-zero/net-zero-bench-marking-and-analysis/natural-gas-carbon-footprint-analysis/

- Carbon intensity for UK domestic natural gas production shown as average 0.18 kg CO2e/kg NG in the UK (converted from kg CO2/boe with the heating value and density of regular NG) and compared to imported Liquified Natural Gas (LNG) from various countries to the UK with average 0.66 and up to 0.81 kg CO2e/kg. Note that imported LNG must always have a significantly higher PCF due to the additional emissions related to transport/processing, liquefaction, LNG tanker shipping and regasification. In practice often a mix of several natural gas sources is used in localized chemical production and therefore the PCF of natural gas varies significantly from country to country and also over time.

#c) Fernandez-Gonzalez et al, CO2 electroreduction: Sustainability analysis of the renewable synthetic natural gas, Int. J. of Greenhouse Gas Control 114 (2022) 103549, https://doi.org/10.1016/j.ijggc.2021.103549; page 4: "The mean CF of NG in Europe is estimated as 0.593 kg CO2e/kg NG and varies from a representative range depending on the country and source within 0.166 to 0.917 kg CO2e/kg NG (Swiss Centre for Life Cycle Inventories, 2020)."

#d) Flannery et al.#; WP22-16 M22; NAICS CODE 325120; Chapter 2; page 3-4; average of cryogenic and PSA process, both based on natural gas (modern, not coal-based; 95 Vol% low purity oxygen - for comparison): 0.15 kg CO2e/kg oxygen; relevant for acetylene production is high purity oxygen using electricity based on natural gas (modern, not coal-based; 99.5 Vol% high purity oxygen): 0.37 kg CO2e/kg oxygen

#e) Belaissaouia et al., Energy Efficiency of Oxygen Enriched Air Production Technologies: Cry-ogeny vs Membranes, Energy Procedia 63 ( 2014 ) 497 - 503, doi: 10.1016/j.egypro.2014.11.054; this publication shows the specific energy consumption in kWh/ton oxygen for state-of-the-art membrane (~95 Vol% low purity) and cryogenic distillation (>99 Vol% high purity) technologies. Fig 4, page 502, clearly shows that the specific energy consumption is rising steeply in the region >98 Vol% and thus high purity oxygen relevant for acetylene synthesis. This is to illustrate the fact, that the PCF of high purity oxygen depends not only on the source of electricity and the air separation technology/efficiency but also significantly on the purity grade of oxygen.

#f) Source: U.S. Energy Information Administration, U.S. electricity net generation and resulting CO2 emissions by fuel in 2021 (https://www.eia.gov/tools/ faqs/faq.php?id=74&t=11), datatable downloaded January 2024

#g) Amgad Elgowainy, Argonne National Laboratory: Presentation at H2IQ webinar, June 15 5 2022, GREET® MODEL FOR HYDROGEN LIFE CYCLE GHG EMISSIONS (slide 8); https://www.energy.gov/sites/default/files/2022-06/hfto-june-h2iqhour-2022-argonne.pdf, downloaded January 2024.

PCF Examples acetylene and synthesis gas: mass-allocation

Three scenarios were calculated for acetylene and synthesis gas industrial production based on the input ratios and the respective Raw Material PCF and energy backpack base data as shown in the tables above.
By using the input and output ratios based on the total value product stream (definitions see above) the resulting PCF values as follows are defined as mass-allocated PCF for the total value product mix acetylene+AOG2(dry) for the co-production of acetylene and synthesis gas via partial combustion of hydrocarbons with an oxidizing agent. This mass-allocated PCF is then identical to the PCF of both products acetylene and synthesis gas (same numbers for both products) because it is a "specific figure" specified in the unit kg CO2e/kg product.

Note: The TfS Guideline mentions on p. 66 "If hydrogen is a co-product allocation by heating value shall be applied because of the low molecular weight of hydrogen. Example: Syngas process that generates CO and hydrogen, both are gases and valuable products. If hydrogen is a co-product in a multi-output process, mass allocation shall not be applied because of the low molecular weight of hydrogen". In the scope of this patent application we do not apply allocation by heating value for simplification purposes - the inventive effect of using pure oxygen supplied from water electrolysis as oxidizing agent is exactly the same with both PCF allocation principles, since only the numbers are changing (e.g. PCF weighted by heating value acetylene vs. synthesis gas - the product with the higher heating value gets a higher PCF while the other co-product's PCF is lowered accordingly) but not the relative impact on the PCF values.

Also for simplification purposes, in the following PCF examples the term "total value product mix acetylene+AOG2(dry)" will be substituted by "acetylene and synthesis gas". Explicitly, this means the terms PCF(mass-allocated) of the total value product mix acetylene+AOG2(dry) and PCF(mass-allocated) of acetylene and synthesis gas are used synonymously. This is feasible because we have chosen mass-allocation for the PCF calculations as explained above.

To summarize, the following boundary conditions have been used (scenario i), ii), iii)):

i) Fossil conventional: Hydrocarbons / Natural Gas is fossil based and the oxidizing agent used in step i) (high purity oxygen) is no oxidizing agent comprising $O_2$ and $H_2$, preferably obtained at least in part by water splitting, but instead conventional cryogenic oxygen obtained by air separation technology is employed. Additionally, steps i) to iv) are carried out on the basis of predominantly fossil energy (comparative example);

ii) According to the present invention: 50% of the oxidizing agent used in step i) is obtained by water electrolysis based on electrical power generated from non-fossil resources (oxidizing agent containing 0.5 Vol% of hydrogen) and 50% of electricity and thermal energy used in steps i) to iv) are based on non-fossil energy;

iii) Best-case according to the present invention: 100% of the oxidizing agent used in step i) is obtained by water electrolysis based on electrical power generated from non-fossil resources (oxidizing agent containing 0.5 Vol% of hydrogen) and electricity and thermal energy used in steps i) to iv) are predominantly based on non-fossil energy; additionally fossil-based natural gas with best-case very low PCF is used in step i) as hydrocarbon.

The results for the total cradle-to-gate product carbon footprint PCF (mass-allocated) of acetylene and synthesis gas are summarized in the following table 2 and plotted in Figure 1 (Fig. 1).

Table 2:

| Acetylene and synthesis gas | Scenario i) Comparative | Scenario ii) | Scenario iii) |
| --- | --- | --- | --- |
| Total Raw Materials | 0.59 | 0.26 | 0.07 |
| Total Energy/Waste | 0.60 | 0.45 | 0.31 |
| Total cradle-to-gate PCF (mass-allocated) | 1.19 | 0.71 | 0.38 |

Fig. 1 shows the results PCF (mass-allocated) of acetylene and synthesis gas.

The black colored part of the colums is the PCF (mass-allocated) of the total raw materials. The white colored part of the colums is the PCF (mass-allocated) of the total energy/waste.

Clearly, the total cradle-to-gate PCF (mass-allocated) of acetylene and synthesis gas in the inventive examples is significantly lower than the fossil-based comparative example. By using purified oxygen supplied from water-electrolysis as oxidizing agent according to the present invention the Total Raw Materials PCF (mass-allocated) is more than halved which can be seen clearly in table 2.

It was found that, the resulting PCF (mass-allocated) of acetylene and synthesis gas does not only highly depend on the actual source and thus the PCF of the hydrocarbons / natural gas, but also on the oxidizing agent / purified oxygen (see ranges of data explained above).

In chemical production practice it is also common to use over time varying mixtures of different raw material sources and suppliers based on current availability and cost development. Therefore, in order to correctly describe the PCF (mass-allocated) of acetylene and synthesis gas according to the invention the following calculation specification formula has been found by the inventors, based on the input ratios for state-of-the art continuous partial combustion production technology with closed water-quench mode.

Said following calculation specification formula is a mathematical correlation, showing the separate contributions of raw materials, energy and waste to the total product carbon footprint (PCF). To be specific, each "summand" of the formula and the resulting (mass-allocated) PCF value have the unit kg CO2e/kg acetylene and synthesis gas:
PCF (mass-allocated) of acetylene and synthesis gas <= 0.283 + 0.847 * PCF(Oxidizing agent) + 0.676 * PCF (Hydrocarbons for synthesis (raw material)) + 0.456 * PCF (Electricity) + 0.441 * PCF (Thermal Energy) + 0.054 * PCF (Heating and flaring fuel)

Whereby the following units must be used so that the PCF(mass-allocated) of acetylene and synthesis gas is given in kg CO2e/kg acetylene and synthesis gas:

- PCF(Oxidizing agent) and PCF(Hydrocarbons for synthesis): kg CO2e/kg Raw Material
- PCF(Electricity): kg CO2e/kWh
- PCF(Thermal Energy): kg CO2e/kg steam
- PCF(Heating and flaring fuel): kg CO2e/kg fuel

Note that the fix contribution of 0.283 kg CO2e/kg acetylene and synthesis gas results from the total waste output ratio (shown as 100% carbon) multiplied with the fixed factor $CO_2$/C molar mass ratio, data sources and explanation see above.

Surprisingly, it can directly be seen in the formula, that the PCF of the oxidizing agent has the greatest contribution to the PCF(mass-allocated) of acetylene and synthesis gas and therefore using oxygen generated by water electrolysis based on electrical power generated from non-fossil resources is the greatest lever to achieve a low PCF acetylene product.

Furthermore, using the material oxygen of water electrolysis as raw material for acetylene production according to the invention results in additionally advantageous synergies: a) the already allocated and inherently scarce renewable electricity for green hydrogen production can be used as well for "green oxygen" low PCF raw material input for acetylene and synthesis gas production. Usually, the valuable oxygen generated by green hydrogen production is released to the atmosphere. Additionally, cryogenic oxygen production coupled with nitrogen production via air separation unit (ASU) technology and related emissions from its high electricity consumption are significantly reduced because the very high oxygen demand for acetylene and synthesis gas production can be covered at least partially by the water electrolysis oxygen co-product. Even on large chemical production cluster sites the demand for nitrogen is generally not so high, that the oxygen demand for a world-scale acetylene plant can be completely covered by ASU since air only contains ~23 wt% of oxygen and thus it's usually a bottleneck requiring additional ASU capacity for state-of-the-art acetylene plants even on large Verbund sites. Therefore, by preferably using the complete material oxygen output from water electrolysis primarily used for green hydrogen, not only the low PCF of the electrolysis oxygen is smartly used but also the total ASU production capacity can be significantly reduced leading to additional savings of emissions (ASU investment and operations with inherently scarce renewable electricity). These effects clearly differentiate the present invention from using ASU oxygen based on partially renewable electricity.

By conducting a detailed acetylene synthesis simulation study comparing ASU oxygen and electrolyzer oxygen the inventors have also surprisingly found completely unexpected effects as shown below.

Example 2: Acetylene synthesis simulation examples

Cryogenic oxygen (oxygen obtained by separation from the atmosphere) and oxygen generated on the anode side of water electrolysis are based on completely different sources (starting materials). Whereas cryogenic oxygen is based on $O_2$ present in ambient air (from the atmosphere), oxygen generated on the anode side of water electrolysis is based on the oxygen present in the water molecules used for water-splitting-electrolysis. The by-product spectrum of other remaining / residual gases (as well as the isotope ratio ($^{18}O/^{16}O$) (see above and below) is therefore distinctively different.

Cryogenic high purity oxygen used for acetylene production consists usually of >99 Vol% O2 and up to ~1 Vol% of inert gas, mainly N2 and small amounts of Ar resulting from imperfect distillation of liquified air.

Oxygen generated on the anode side of water electrolysis is known to show a substantially different composition compared to ASU oxygen. First of all, nitrogen and argon as found in ASU oxygen is much lower due to water electrolyzers requiring highly purified ("ultra-pure") water as raw material in order to prevent fouling / scaling and also to remove CO2 which can represent a significant ionic load. Various UPW technologies (ultrapure treatment and purification systems) for electrolyzer loop and makeup streams are state-of-the art, such as RO (reverse osmosis) and degassing among other water treatment / "polishing" steps. This results usually N2 and Ar content of electrolyzer gas in the ppmv range and thus approximately two orders of magnitude lower inert gas content compared to ASU oxygen.

However, on the other hand, electrolysis oxygen generally inherently contains significant amounts of hydrogen largely depending on the electrolyzer technology and the applied gas treatment setup. The presence of hydrogen in the oxygen gas can be explained by the high mobility of hydrogen and the remaining gas permeability of the electrolysis membranes. Due to safety requirements (explosive range begins at approx. 4 Vol% H2 in O2 at 1 atm, depending on temperature and pressure) the H2 concentration must be strictly controlled, usually below 2 Vol%. In operational practice, the H2 content in the electrolysis oxygen is especially depending on the electrolyzer load - at lower / partial load the H2 concentration usually increases depending on the supplier, choice of membrane material and thickness and its quality as well as the electrolyzer running-time usually leading to increased porosity over time. Last-but-not least electrolyzer operational pressure and temperature play a significant role in hydrogen crossover effects. Also the influence of electrolyzer cell load is especially relevant for cyclical green electricity production e.g from solar and wind power. Depressing the H2-content in the oxygen gas of current state-of-the-art electrolysis technology significantly below 0.1 Vol% is very difficult and leads to unacceptably high costs for green hydrogen and oxygen commodity volume production as required for acetylene synthesis.

Compared to using ASU oxygen, the presence of hydrogen in electrolysis oxygen has prevented the person skilled in the art in the past from using said electrolysis oxygen in the acetylene synthesis process. Reasons therefore are:

- The process requiring preheated (e.g. 600°C) purified oxygen as raw material feed which leads to the requirement to include the H2-content in electrolysis oxygen into the process safety concept (e.g. avoiding explosive regimes and preventing risk of back-firing from the mixing/burner block).
- Additionally, the low heating-value of hydrogen compared to the hydrocarbon feed was expected to cool the flame in the partial oxidation reactor setup thereby leading to increased oxygen consumption followed by reduced hydrocarbon conversion to acetylene and thereby depressing the acetylene yield and/or increasing the soot generation.

It is well known, that acetylene yield and soot generation can be steered with the hydrocarbon/oxygen raw material ratio, usually controlled via the "oxygen ratio" (lambda) defined as the ratio of the amount of oxygen actually present to the stoichiometrically necessary amount of oxygen required for the full combustion of the hydrocarbon feedstock (see the related art mentioned in the introduction of the present application). However, using electrolysis oxygen with a significant content of hydrogen was considered to be prohibitive for acetylene production because of depressing yield and simultaneously increasing soot generation effects. Even if acetylene yield loss could be compensated by adjusting the oxygen ratio a significantly higher soot generation was to be expected by persons skilled in the art when using electrolysis oxygen in acetylene synthesis.

Further, water may be present in the oxygen gas of water electrolysis, generally stemming from the vapor-pressure of the inherently water saturated gas at the operational temperature/pressure on both the anode and cathode side. The order of magnitude could be comparable to saturated air holding ~17 g H2O at 20°C and already ~195 g H2O per kg dry air at 70°C (100% rel. humidity, 1 atm) which are typical electrolyzer operating conditions. Therefore, not only the hydrogen produced by electrolysis is usually dried (driven by the H2 specification limiting the water content) but also the electrolysis oxygen gas is preferably dried. Usually in a first stage heat exchangers operated with cooling and/or chilled water are used to condense water from the product gas. For maximum drying requirements additionally e.g. (multiple) absorber beds filled with a regenerative desiccant can be used in water electrolysis operational practice. As an example the electrolyzer brochure PD-0600-0125 Rev D by Nel ASA "Alkaline and Proton PEM Electrolysers" shows a typical gas dryer setup for standard large-scale industrial PEM (Proton Exchange Membrane) and AEL (Alkaline Electrolysis) electrolyzers (https://nelhydrogen.com/wp-content/uploads/2024/01/Electrolysers-Brochure-Rev-D-1.pdf, downloaded January 2024). To sum up, the water content in electrolysis gases can be lowered and adjusted as per gas purity requirements but it comes at the price for investment and higher operational costs. For example, a specification <0.1 Vol% H2O is of course much easier to achieve than <10 ppmv H2O requiring large adsorption towers which also increase energy consumption.

Table 3 shows exemplary and representative absolute oxygen feedstock compositions showing the main constituents O2, H2O, H2 and N2 for several commercial ASU and electrolysis technologies. Small quantities of other impurities may be present as well, depending on the raw material supply and process conditions: e.g. CO, CO2, hydrocarbons (often summarized as THC, total hydrocarbon content) as well as various noble and trace gases. Such small impurities are neglected in this study due to the inferior contributions to acetylene synthesis at levels in the ppmv-range.

Here we consider standard cryogenic ASU high purity oxygen usually used for acetylene production (comparative examples) in contrast to the oxygen gas supplied by two major standard electrolyzer technologies (inventive examples). The practical effect of running ASU as well as electrolyzers at different load is clearly shown: ASU oxygen can contain more or less inert gases (mainly N2; Ar and other trace gases are not considered here) while the water content is very low (<10 ppmv) and hydrogen is clearly not detectable (<1 ppmv). On the other hand electrolyzer oxygen always shows a significant H2-content up to the permissible safety conceptual limit (e.g. 2 Vol%). Additionally, a variable water content may be present in the electrolyzer oxygen. Electrolyzer gas humidity can be adjusted by adequately designed gas drying units and its setpoint (see above). Inert gas presence such as N2 is usually - if present at all - at least an order of magnitude lower in electrolyzer oxygen compared to ASU.

Table 3

| Technology study scenarios | | Absolute gas composition (Vol%) | | | |
|---|---|---|---|---|---|
| | | O2 | H2O | H2 | N2 |
| **Comparative Examples** | | | | | |
| C1 | ASU Oxygen - 50% load | 99.74 | < 10 ppmv | < 1 ppmv | 0.23 |
| C2 | ASU Oxygen - 90% load | 99.49 | < 10 ppmv | < 1 ppmv | 0.44 |
| | | | | | |
| **Inventive Examples** | | | | | |
| A1 | PEM - CP=30%, GD=0.1 | 99.20 | 0.1 | 0.7 | < 10 ppmv |
| A2 | PEM - CP=50%, GD=0.1 | 99.40 | 0.1 | 0.5 | < 10 ppmv |
| A3 | PEM - CP=90%, GD=0.1 | 99.65 | 0.1 | 0.25 | < 10 ppmv |
| | | | | | |
| A4 | PEM - CP=30%, GD=0.2 | 99.10 | 0.2 | 0.7 | < 10 ppmv |
| A5 | PEM - CP=50%, GD=0.25 | 99.25 | 0.25 | 0.5 | < 10 ppmv |
| A6 | PEM - CP=90%, GD=0.3 | 99.45 | 0.3 | 0.25 | < 10 ppmv |
| | | | | | |
| A7 | AEL - CP=30%, GD=0.2 | 98.60 | 0.2 | 1.2 | < 10 ppmv |
| A8 | AEL - CP=50%, GD=0.25 | 98.95 | 0.25 | 0.8 | < 10 ppmv |
| A9 | AEL - CP=90%, GD=0.3 | 99.05 | 0.3 | 0.65 | < 10 ppmv |

Notes and references

- ASU = Air Separation Unit; PEM = Proton Exchange Membrane electrolyzer; AEL = Alkaline electrolyzer; CP = percentage of electrical current based on maximum design current (setpoint of electrolyzer cell-load controller, e.g. Ampere %); GD = gas humidity setpoint of electrolyzer oxygen gas drying unit
- ASU oxygen purity ranges: SERVOMEX brochure AB15 Rev.0 04/11 E "Servomex Total Solution for Gas Analysis on Cryogenic Air Separation Plants", https://www.dastec-srl.com.uy/uploads/aplicacion-downloads/migracion/2/104.pdf?v154 downloaded January 2024
- Oxygen purity of various commercial electrolyzers: Zeng, K., & Zhang, D. (2010). Recent progress in alkaline water electrolysis for hydrogen production and applications. Progress in Energy and Combustion Science: an international review journal, 36, 307-326. https://doi.org/10.1016/j.pecs.2009.11.002
- A World Of Energy, Vincent Knop, "Part load operation of alkaline electrolysers", published 12 March 2023, https://www.awoe.net/Water-Electrolysis-Alkaline-Part-Load.html as of January 2024

In order to investigate the quantitative impact of using the very different oxygen gas feed compositions for industrial acetylene synthesis, numerical simulations of freely-propagating premixed laminar flames were performed using the Python version of Cantera (version 3.0.0) (https://cantera.org/documentation/release_notes/v3.0.0.html). All calculations were performed based on US 5,824,834 Example 1 with 6,400 $Nm^3$/h hydrocarbons (methane) and 4,200 $Nm^3$/h oxidizer agent feed equal to an oxygen ratio of lambda = 0.323 as typical operating point with high acetylene and synthesis gas yields and low soot generation. Note: In the context of the simulations, oxygen ratio is defined as the molar ratio of the amount of oxygen actually present to the stoichiometrically necessary amount of oxygen required for the full combustion of the feedstocks, whereby feedstocks in this respect are hydrocarbons and explicitly also hydrogen, if applicable. A preheating temperature of 600°C and a pressure of 1 atm was chosen for all examples and the reaction mechanism used was the CalTech2.3 mechanism (The FORCE: Cal-techMech), which has been developed and validated for detailed C2-chemistry including the formation of carbon black precursors such as $C2H2$ (acetylene) and PAH (poly-aromatic hydrocarbons). Sentko et al. showed that the described mechanism reproduces the profiles of species concentrations, especially hydrocarbons, in fuel-rich premix flames very well (Experimental investigation of synthesis gas production in fuel-rich oxy-fuel methane flames, Fuel 2022, 317, 123452; https://doi.org/10.1016/j.fuel.2022.123452).

The simulations allow calculation of key output parameters such as acetylene C2H2 yield (mol-%) based on the hydrocarbon feed, soot generation (here based on the precursor Pyrene generation) as well as the CO/CO2-ratio relevant for the synthesis gas output. For the example evaluations a fixed distance of 14.0625 mm was chosen to simulate a quench near the C2H2 peak concentration and ensure comparability of the numerical results.

As a last step, all main parameters (O2, H2O, H2 and N2-content in the oxygen feed) as well as the oxygen ratio (see definition above) have been studied separately in order to clearly show the quantitative impacts without any cross-effects.

| Parameter study scenarios | | Oxygen ratio (-) | Absolute gas composition (Vol%) | | | |
|---|---|---|---|---|---|---|
| | | | O2 | H2O | H2 | N2 |
| Comparative Examples | | | | | | |
| C3 | Base Case 1 | 0.3230 | 100 | - | - | - |
| C4 | Water 1 Vol% | 0.3230 | 99.0 | 1.0 | - | - |
| C5 | Water 2 Vol% | 0.3230 | 98.0 | 2.0 | - | - |
| C6 | Nitrogen 1 Vol% | 0.3230 | 99.0 | - | - | 1.0 |
| C7 | Nitrogen 2 Vol% | 0.3230 | 98.0 | - | - | 2.0 |
| C8 | Oxygen ratio 0.3225 | 0.3225 | 100 | - | - | - |
| C9 | Oxygen ratio 0.3235 | 0.3235 | 100 | - | - | - |
| | | | | | | |
| Inventive Examples | | | | | | |
| A10 | H2 0.1 Vol% | 0.3230 | 99.9 | - | 0.1 | - |
| A11 | H2 0.5 Vol% | 0.3230 | 99.5 | - | 0.5 | - |
| A12 | H2 1.0 Vol% | 0.3230 | 99.0 | - | 1.0 | - |
| A13 | H2 1.5 Vol% | 0.3230 | 98.5 | - | 1.5 | - |
| A14 | H2 2.0 Vol% | 0.3230 | 98.0 | - | 2.0 | - |

All simulation results have been normalized to US 5,824,834 Example 1 (Base Case 1) so that the impact of changed feed compositions is clearly visible with quantitative positive and negative effects. The resulting data is shown in tables 4 and 5.

Table 4

| Technology study scenarios | | Yield C2H2 (%*) | Pyrene/Soot (%*) | CO/CO2-ratio (%*) |
|---|---|---|---|---|
| Comparative Examples | | | | |
| C1 | ASU Oxygen - 50% load | 100.0 | 101.0 | 100.0 |
| C2 | ASU Oxygen - 90% load | 100.0 | 102.1 | 99.9 |
| | | | | |
| Inventive Examples | | | | |
| A1 | PEM - CL=30%, GD=0.1 | 100.2 | 96.3 | 100.1 |
| A2 | PEM - CL=50%, GD=0.1 | 100.1 | 97.5 | 100.0 |
| A3 | PEM - CL=90%, GD=0.1 | 100.0 | 98.9 | 100.0 |
| | | | | |
| A4 | PEM - CL=30%, GD=0.2 | 100.1 | 96.7 | 100.0 |
| A5 | PEM - CL=50%, GD=0.25 | 100.2 | 99.1 | 100.0 |
| A6 | PEM - CL=90%, GD=0.3 | 100.0 | 99.7 | 99.8 |

(continued)

| Inventive Examples | | | | |
|---|---|---|---|---|
| | | | | |
| A7 | AEL - CL=30%, GD=0.2 | 100.3 | 93.8 | 100.1 |
| A8 | AEL - CL=50%, GD=0.25 | 100.1 | 96.3 | 100.0 |
| A9 | AEL - CL=90%, GD=0.3 | 100.1 | 97.3 | 99.9 |
| * All results normalized to Base Case 1 = 100% | | | | |

Table 5

| Parameter study scenarios | | Yield C2H2 (%*) | Pyrene/Soot (%*) | CO/CO2-ratio (%*) |
|---|---|---|---|---|
| Comparative Examples | | | | |
| C3 | Base Case 1 | 100.0 | 100.0 | 100.0 |
| C4 | Water 1 Vol% | 99.7 | 104.2 | 99.1 |
| C5 | Water 2 Vol% | 99.4 | 108.9 | 98.2 |
| C6 | Nitrogen 1 Vol% | 99.9 | 104.2 | 99.8 |
| C7 | Nitrogen 2 Vol% | 99.9 | 109.0 | 99.6 |
| C8 | Oxygen ratio 0.3225 | 100.6 | 109.8 | 99.8 |
| C9 | Oxygen ratio 0.3235 | 99.5 | 91.7 | 100.1 |
| | | | | |
| Inventive Examples | | | | |
| A10 | H2 0.1 Vol% | 100.0 | 99.4 | 100.0 |
| A11 | H2 0.5 Vol% | 100.1 | 97.1 | 100.1 |
| A12 | H2 1.0 Vol% | 100.3 | 94.2 | 100.2 |
| A13 | H2 1.5 Vol% | 100.4 | 91.4 | 100.3 |
| A14 | H2 2.0 Vol% | 100.6 | 88.6 | 100.5 |
| * All results normalized to Base Case 1 = 100% | | | | |

Completely surprisingly, the study clearly proves that hydrogen in the oxidizing agent feed actually shows a synergetic positive effect on a) acetylene yield (increase, leading to raw material cost reduction and higher daily acetylene output) b) soot generation (reduction, leading to lower waste generation and longer running-time due to fouling-reduction downstream of the flame reactor) and c) CO/CO2-ratio (increase, leading to higher output of synthesis gas value product and less CO2 waste generation).

The study also shows the well-known effect for conventional acetylene production, that the oxygen ratio is a very sensitive parameter acting on both acetylene yield and soot generation with clear opposite effects (assuming all other operating parameters are constant): increasing yield always results in higher soot generation, as shown with the example series C8, C3, C9. Therefore in examples A10 to A14 the hydrogen concentration in the oxidizing agent was increased while keeping the oxygen ratio constant. Because hydrogen is here considered as additional combustible feedstock on top of the hydrocarbons, this means that the effective total oxidizing agent feed is increased slightly higher compensating the increasing H2-content compared to the classical oxygen ratio control which only considers the combustible hydrocarbons. By using this new control strategy the surprisingly synergetic positive effect of using an oxidizing agent comprising hat least 1 ppmv of hydrogen beside oxygen, preferably electrolysis oxygen, for acetylene synthesis was found in this study. The effect is also clearly visible in the comparative study of realistic ASU oxygen (examples C1, C2) and electrolyzer oxygen feedstocks (examples A1 - A9).

The study also clearly shows, as expected, that increasing N2 content in the oxidizing agent feed shows a negative effect on all 3 target paramters acetylene yield, soot generation and CO/CO2-ratio (examples C3, C6, C7) without being bound to a theory caused by cooling of the flame and dilution / loss of residence time. The same negative effect, as expected, was found for increasing water content in the oxidizing agent feed (examples C3, C4, C5). This explains why in the electrolyzer examples with low hydrogen and high water content (e.g. A6) the acetylene yield is not improved compared to the base case. However, surprisingly, the yield improving effect of hydrogen is so strong that even a moisture content above 0.1 Vol% can still deliver similar yield as with ASU oxygen and no significant increase of soot generation or loss of CO/CO2-ratio.

The maximum positive and inventive effect for using electrolyzer oxygen for acetylene synthesis can be achieved by providing a maximum H2-content (up to ~2 Vol% (20000 ppmv) H2, as high as possible = permitted by the safety limits) combined with the lowest practically achievable and/or cost-optimized oxygen gas humidity. Based on this study, such H2-containing oxidizing agent feeds, preferably coming from oxygen gas obtained by water electrolysis will surprisingly not depress acetylene yield and not increase soot generation as well as not reduce the CO/CO2-ratio but improve all 3 parameters compared to using conventional ASU oxidizing agent feed. The quantitative effects in Tables 4 and 5 may seem small at first sight due to the relatively small H2-contents but are still significant since +0.5% absolute higher acetylene yield means +500 tons/a higher output for a world-scale 100 kt/a acetylene plant and significantly lower specific hydrocarbon raw material costs expressed in €/t acetylene as well as for the synthesis gas co-product manufacturing costs. Additionally, the quality of synthesis gas as valuable co-product increases due to lower CO2 and higher CO-content and waste generation is reduced which in turn also leads to a measurable PCF-reduction not only caused by the improved raw material yield.

Example 3: Acetylene and Synthesis Gas Production using Water Electrolysis vs. ASU Oxygen

Oxygen gas from different industrial sources, prepared via ASU and water electrolysis technology under different conditions have been analyzed for its absolute gas composition with varying content of O2, H2O, H2 and N2 as well as $\delta$18O istopic ratio.

In this context $\delta$18O means the ratio of the stable isotopes oxygen-18 (180) and oxygen-16 (160) in the so called delta-notation versus a reference standard material. The definition of $\delta$18O is, in "per mil" (‰, parts per thousand):

$$\delta^{18}\mathrm{O} = \left( \frac{\left(\frac{^{18}\mathrm{O}}{^{16}\mathrm{O}}\right)_{\mathrm{sample}}}{\left(\frac{^{18}\mathrm{O}}{^{16}\mathrm{O}}\right)_{\mathrm{standard}}} - 1 \right) \times 1000\ ‰$$

As standard material reference with known isotopic composition Vienna Standard Mean Ocean Water (VSMOW) was chosen. Therefore, $\delta$18O vs. VSMOW shows the difference of the sample's 18O/16O-ratio versus VSMOW. For example, $\delta$18O = +10 per mil means that the sample has a 10 per mil higher 18O/16O isotope-ratio than VSMOW thus the sample is accordingly enriched with the heavy oxygen isotope 180. On the other hand, negative $\delta$18O values mean, that the respective sample is depleted in 180 versus VSMOW.

Such delta isotopic oxygen ratio data is commonly used for example in geochemistry and climatology for example as a measure of the temperature of precipitation or of mineral interactions in groundwater as well as isotopic fractionation processes. Mass spectrometry is widely used as analytical method with high precision and reproducibility in the order of magnitude of at least (+-) 0.2 per mil.

In the context of this invention water, air, oxygen and synthesis gas have been analyzed for $\delta$18O. Due to slightly different liquid and gas sample preparation requirements and especially due to the different chemical composition the analytical methods differ accordingly.

Therefore it was chosen to use dedicated analytical methods for water, air, oxygen and synthesis gas samples analyzing particularly the H2O, O2 and CO species in those samples. In particular, for water samples a CO2/H2O-equilibrium based method was used, while for air the analysis was conducted with reference O2 gas and for synthesis gas samples a method was chosen which extracts CO and converts CO to CO2 with Schütze reagent. All methods use Isotope Ratio Mass Spectrometry (IRMS) technology and the procedures are described in detail in the following references:

1) Water liquid samples: Martins et al., Stable isotopes of oxygen and hydrogen in water: analytical method evaluation and the determination of $\delta$ 180 and $\delta$ 2H on a control sample, Quim. Nova, Vol. 46, No. 4, 343-350, 2023 (http://dx.doi.org/10.21577/0100-4042.20230021)

2) Air and oxygen gas samples: Wassenaar et al., An On-Line Technique for the Determination of the $\delta$ 18O and $\delta$ 17O of Gaseous and Dissolved Oxygen, Anal. Chem. 1999, 71, 4965-4968 (DOI: 10.1021/ac9903961)

3) Synthesis gas samples: Pathirana et al., An analytical system for studying the stable isotopes of carbon monoxide using continuous flow-isotope ratio mass spectrometry (CF-IRMS), Atmos. Meas. Tech. Discuss., 8, 2067-2092, 2015 (DOI: 10.5194/amtd-8-2067-2015)

The gas analytical results of the industrial oxygen sources used in this example are shown in table 6. The hydrogen content varies with different electrolysis technology and employed load conditions while the water content (humidity) was regulated with the oxygen gas drying unit.

Clearly, the isotope analysis shows a pronounced difference between ASU oxygen (>+23 per mil) vs. electrolysis oxygen <+2 per mil - one order of magnitude. A slight reduction of $\delta$ 18O in the oxygen gas compared to the electrolysis water was found depending on the conditions employed. The $\delta$ 18O values for the gas mixtures are in line with the composition - the more ASU oxygen is contained in the mixture, the closer the $\delta$ 18O value get to the ambient air used for cryogenic air separation.

Natural gas with constant raw material composition and oxygen supplied via pipeline from the 3 different sources (cryogenic ASU oxygen, PEM electrolysis and AEL electrolysis oxygen operated with different settings as described below) have been used for acetylene and synthesis gas production as described in US 5,824,834 Example 1.

The reactant gases were separately heated to 600°C, intimately mixed in the mixing zone of the burner and brought to reaction after passing through a diffuser and the burner block. After a reaction time of a few milliseconds the acetylene containing cracked crack gases were quenched with water to a temperature of 80 °C. The products acetylene and synthesis gas were obtained in a normal manner by fractionated absorption and subsequent desorption with aid of a suitable solvent. The soot formed as a by-product was obtained from the quench and cooling water which was removed from the closed quench or cooling water circuit. The exact experimental conditions and results of the experiments are summarized in table 7.

Clearly, the experimental results show that by using oxygen comprising a small amount of hydrogen (oxygen from water electrolysis (example A30 to A33)) compared to conventional oxygen from air separation (C30, C31) the daily acetylene output does not decrease but tenden-tially a small increase of acetylene yield is found. The best results were found with >1 Vol% hydrogen concentration in the oxidizing agent feed giving approx. 0.3% higher acetylene output which was found statistically relevant over the campaign results. Moreover, a pronounced effect in reduced soot production was found while in conventional acetylene production higher yield/acetylene always results in higher soot production, here the opposite was found. The examples with mixtures of cryogenic and electrolysis oxygen show, that it is also possible to employ a gas mixed feed - the higher the content of oxygen comprising a small amount of hydrogen (here: electrolysis oxygen content), the better.

The isotope analysis of the synthesis gas shows approximately the same $\delta$ 18O figures as the oxygen employed in the experiments. This means, that the pronounced difference between ASU oxygen and electrolysis oxygen employed - almost one order of magnitude - is also visible in the $\delta$ 18O values for the synthesis gas produced via the inventive acetylene process.

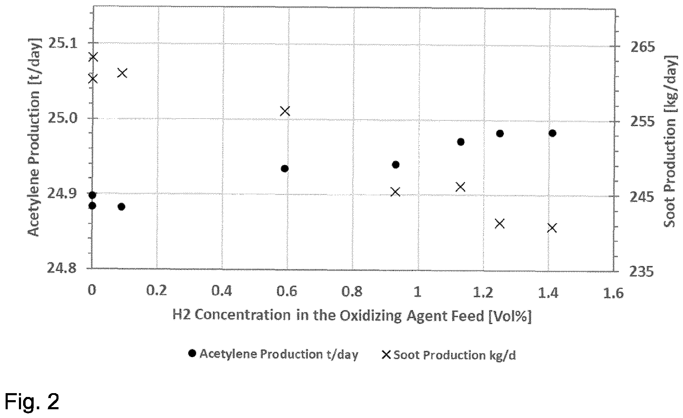

Figure 2 shows a plot of the acetylene production and soot production versus. the absolute hydrogen concentration in the oxidizing agent feed. It can be seen that the hydrogen content in the oxygen drives both, increased acetylene output and reduced soot production. The effects are clearly demonstrated in this campaign production under steady-state conditions with varying oxidizing agent feeds.

Table 6

| | | Isotope Analysis a) | Absolute gas composition (Vol%) d) | | | |
|---|---|---|---|---|---|---|
| | | $\delta$18O vs VSMOW [per mil = ‰] | O2 | H2O | H2 | N2 |
| Comparative Examples: Air separation b) | | | | | | |
| C20 | ASU Oxygen-- 46% load | +23.4 | 99.6 | < 10 ppmv | <1 ppmv | 0.20 |
| C21 | ASU Oxygen-- 92% load | +23.6 | 99.4 | < 10 ppmv | <1 ppmv | 0.45 |
| | b) Cryogenic air separation unit operated with ambient air with $\delta$18O +23.8 per mil | | | | | |
| | | | | | | |

(continued)

| Inventive Examples: Electrolysis [c] | | | | | | |
|---|---|---|---|---|---|---|
| A20 | PEM Oxygen-- CL=0.4V, CP=34% | +1.9 | 98.6 | < 0.1 | 1.13 | < 10 ppmv |
| A21 | PEM Oxygen-- CL=0.6V, CP=79% | +1.6 | 98.8 | < 0.1 | 0.93 | < 10 ppmv |
| A22 | AEL Oxygen-- CL=0.4V, CP=51% | +1.3 | 98.4 | < 0.1 | 1.41 | < 10 ppmv |
| A23 | AEL Oxygen-- CL=0.7V, CP=91% | -1.1 | 98.5 | < 0.1 | 1.25 | < 10 ppmv |
| A24 | 50:50 Vol% Oxygen mix A23 + C21 | +11.5 | 99.0 | < 500 ppmv | 0.59 | 0.21 |
| A25 | 10:90 Vol% Oxygen mix A21 + C20 | +20.9 | 99.5 | < 100 ppmv | 0.09 | 0.19 |
| | a) Analytical methods used depending on sample as described above: all figures are total campaign averages for steady-state continuous production conditions<br><br>c) Electrolysis using Demin Water with $\delta$18O +2.1 per mil CL = approximate current load / Überspannung; CP = percentage of electrical current based on maximum design current (setpoint of electrolyzer cell-load controller, e.g. Ampere %); Humidity H2O content adjusted via setpoint of the oxygen gas drying unit<br><br>d) HORIBA EMGA-Pro Oxygen/Nitrogen/Hydrogen Analyzer with NDIR detector | | | | | | |

Table 7

| | Acetylene and Synthesis Gas Production [e] | | | | | | | |
|---|---|---|---|---|---|---|---|---|
| | Comparative Examples: Oxygen from Air separation | | Inventive Examples: Oxygen from Water Electrolysis | | | | | |
| | C30 | C31 | A30 | A31 | A32 | A33 | A34 | A35 |
| Natural Gas Feed [Nm$^3$/h] | 6,500 | 6,500 | 6,500 | 6,500 | 6,500 | 6,500 | 6,500 | 6,500 |
| Source of Oxygen | from C20 | from C21 | from A20 | from A21 | from A22 | from A23 | from A24 | from A25 |
| Oxygen Feed [Nm$^3$/h] | 4,200 | 4,200 | 4,208 | 4,206 | 4,210 | 4,209 | 4,204 | 4,201 |
| Oxygen ratio [-] | 0.323 | 0.323 | 0.323 | 0.323 | 0.323 | 0.323 | 0.323 | 0.323 |
| Acetylene Production [t/day] | 24.85 | 24.83 | 24.92 | 24.89 | 24.93 | 24.93 | 24.88 | 24.83 |
| Soot Production [kg/day] | 260 | 263 | 246 | 245 | 241 | 241 | 256 | 261 |
| Synthesis Gas [Nm$^3$ per day] | 310,100 | 310,000 | 309,800 | 309,700 | 309,700 | 309,600 | 309,800 | 310,200 |

(continued)

| | Acetylene and Synthesis Gas Production [e] | | | | | | | |
|---|---|---|---|---|---|---|---|---|
| | Comparative Examples: Oxygen from Air separation | | Inventive Examples: Oxygen from Water Electrolysis | | | | | |
| Synthesis Gas Isotope Analysis: $\delta 18O$ vs VSMOW [per mil = ‰] [a] | +23.2 | +23.1 | +1.9 | +1.7 | +1.2 | -1.3 | 11.9 | 21.1 |

[a] Analytical methods used depending on sample as described above: all figures are total campaign averages for steady-state continuous production conditions

[e] Process conditions refer to US 5,824,834 Example 1: Closed water quench acetylene synthesis via partial oxidation of natural gas (98 Vol% CH4); separately pre-heated 600°C reactant gases intimately mixed and brought to reaction after passing through a diffuser and the burner block and subsequently quenched with water to a temperature of 80°C; Feed flow control concept applied: Oxygen feed equipped with online hydrogen gas analysis sensor and DCS control using natural gas feed as reference value and oxygen feed running with ratio-control keeping the "oxygen ratio" constant to setpoint 0.323. "Oxygen ratio" means the ratio of the molar amount of oxygen actually present to the stoichiometrically necessary amount of oxygen required for the full combustion of the feedstocks, whereby feedstocks in this respect are hydrocarbons and hydrogen.

**Claims**

1. Process for preparing acetylene and/or synthesis gas by partial oxidation of hydrocarbons with an oxidizing agent, comprising the steps:

   i) Mixing the hydrocarbons and the oxidizing agent, whereby a mixture of the hydrocarbons and the oxidizing agent is obtained;
   ii) Igniting the mixture of the hydrocarbons and the oxidizing agent in a burner, whereby the partial oxidation of the hydrocarbons takes place and a first cracking gas stream comprising acetylene is obtained;
   iii) Cooling the first cracking gas stream, whereby a second cracking gas stream comprising acetylene is obtained; and
   iv) Extracting the acetylene from the second cracking gas stream, wherein acetylene and/or synthesis gas are obtained;

   wherein the oxidizing agent comprises $O_2$ and $H_2$, preferably at least 1 ppmv of $H_2$, based on the total volume of the oxidizing agent.

2. The process according to claim 1, wherein the oxidizing agent is obtained at least in part by water splitting, preferably by electrolysis, the water splitting, preferably the electrolysis, preferably using energy generated at least in part from non-fossil resources.

3. The process according to claim 1 or 2, wherein the hydrocarbons and the oxidizing agent are preheated separately before the mixing in step i).

4. The process according to any one of claims 1 to 3, wherein the oxygen ratio $\lambda$ in the mixture in step i) is from $\lambda>0.25$ to $\lambda<0.34$, preferably from $\lambda>0.28$ to $\lambda<0.33$.

5. The process according to any one of claims 1 to 4, wherein the hydrocarbons in step i) are selected from the group consisting of natural gas, methane, LPG, naphtha and mixtures thereof, preferably selected from the group consisting of natural gas, methane, LPG, and mixtures thereof, more preferably natural gas and methane.

6. The process according to any one of claims 1 to 5, wherein the second cracking gas stream comprises 5 to 20 Vol% (based on the dry cracking gas stream / without water) of acetylene and synthesis gas comprising hydrogen, CO, CO2 and CH4.

7. The process according to any one of claims 1 to 6, wherein the acetylene is extracted from the second cracking gas stream with NMP, DMF, kerosene, octane, methanol, ammonia, acetone or mixtures thereof.

8. Cracking gas stream obtainable by steps i), ii) and iii) of the process according to any one of claims 1 to 7 and 22.

9. Acetylene obtainable by the process according to any one of claims 1 to 7 and 22.

10. Acetylene having a total cradle to gate mass-allocated product carbon footprint of < 1.1 kg CO2e/kg acetylene, preferably < 1.0 kg CO2e/kg acetylene, more preferably < 0.9 kg CO2e/kg acetylene.

11. Synthesis gas obtainable by the process according to any one of claims 1 to 7 and 22.

12. Synthesis gas comprising hydrogen, CO, CO2 and CH4, wherein the synthesis gas has a $\delta$18O value of < 22 ‰, preferably, < 20 ‰, more preferably < 18 ‰, referred to the international standard VSMOW, preferably synthesis gas according to claim 11.

13. Use of an oxidizing agent comprising $O_2$ and $H_2$, preferably obtained at least in part by water splitting, preferably by electrolysis, the water splitting, preferably the electrolysis, preferably using energy generated at least in part from non-fossil resources for the preparation of acetylene and synthesis gas.

14. Use of the acetylene according to claim 9 or 10 or obtained from the acetylene obtained by the process according to any one of claims 1 to 7 and 22 for the preparation of butynediol, butanediol, butenediol, polybutylene terephthalate (PBT), polybutylene adipate terephthalate (PBAT), tetrahydrofurane (THF), polytetrahydrofurane (polyTHF), polyester-based thermoplastic polyurethanes (TPUs), polyether-based TPUs, gamma-butyrolactone, pyrrolidine, vinylpyrrolidone, polyvinylpyrrolidone, N-methylpyrrolidone, vinyl ether, polyvinyl ether, terpenes and downstream products thereof; and in welding processes and cutting of metals.

15. A process for preparing 1,4-butanediol comprising the following steps:

   (a) reacting acetylene with formaldehyde in the presence of a catalyst, whereby a reaction mixture containing 2-butyne-1,4-diol is obtained;
   (b) hydrogenating the 2-butyne-1,4-diol, whereby a reaction mixture containing 1,4-butanediol is obtained; and
   (c) obtionally isolating 1,4-butanediol from the reaction mixture, preferably by distillation.

   wherein the acetylene in step (a) is at least in part the acetylene according to claim 9 or 10 or obtained from the acetylene obtained by the process according to any one of claims 1 to 7 and 22.

16. Use of the synthesis gas according to claim 11 or 12 or the synthesis gas obtained by the process according to any one of claims 1 to 7 or 22 for the preparation of methanol, fuel applications, formaldehyde, acetic acid, olefins, sodium methylate, methylation products, dimethylterephthalate, methylamine, methyl mercaptane, polyoxymethylene, butynediol, methylendiphenyldiisocyanate, neopentylglycol, phenol formaldehyde resins, urea-condensation resins, melamine resins, acetone resins, chloroacetic acid, acetic acid ester, methylisipropylketone, acetic acid anhydride, dimethylsulfide, methanesulfonic acid and downstream products.

17. A process for preparing methanol from a synthesis gas comprising:

   Conversion of said synthesis gas on a copper-containing catalyst,
   wherein the synthesis gas is at least in part the synthesis gas according to claim 11 or 12 or the synthesis gas obtained by the process according to any one of claims 1 to 7 or 22.

18. A process for preparing formaldehyde from a synthesis gas comprising:
   oxidation of the methanol obtained in the process according to claim 17 to formaldehyde with oxygen, air or a mixture thereof in the presence of a catalyst.

19. A process for preparing 1,4-butanediol according to claim 15, wherein the formaldehyde in step (a) is at least in part the obtained by the process according to claim 18.

20. Use of the $\delta^{18}O$ value in oxygen and downstream compounds based on oxygen for tracing the origin of preparation of oxygen and downstream compounds based on oxygen, wherein the compounds are preferably synthesis gas and downstream products based on synthesis gas, like methanol, fuel applications, formaldehyde, acetic acid, olefins, sodium methylate, methylation products, dimethylterephthalate, methylamine, methyl mercaptane, polyoxymethylene, butynediol, methylendiphenyldiisocyanate, neopentylglycol, phenol formaldehyde resins, urea-condensation resins, melamine resins, acetone resins, chloroacetic acid, acetic acid ester, methylisipropylketone, acetic acid anhydride, dimethylsulfide, methanesulfonic acid and downstream products.

21. A process for tracing the origin of preparation of oxygen and downstream compounds based on oxygen by determining the $\delta^{18}O$ value in oxygen and downstream compounds based on oxygen, wherein the compounds are preferably synthesis gas and downstream products based on synthesis gas, like methanol, fuel applications, formaldehyde, acetic acid, olefins, sodium methylate, methylation products, dimethylterephthalate, methylamine, methyl mercaptane, polyoxymethylene, butynediol, methylendiphenyldiisocyanate, neopentylglycol, phenol formaldehyde resins, urea-condensation resins, melamine resins, acetone resins, chloroacetic acid, acetic acid ester, methylisipropylketone, acetic acid anhydride, dimethylsulfide, methanesulfonic acid and downstream products.

22. Process for preparing acetylene and/or synthesis gas by partial oxidation of hydrocarbons with an oxidizing agent, comprising the steps:

   i) Mixing the hydrocarbons and the oxidizing agent, whereby a mixture of the hydrocarbons and the oxidizing agent is obtained;
   ii) Igniting the mixture of the hydrocarbons and the oxidizing agent in a burner, whereby the partial oxidation of the hydrocarbons takes place and a first cracking gas stream comprising acetylene is obtained;
   iii) Cooling the first cracking gas stream, whereby a second cracking gas stream comprising acetylene is obtained; and
   iv) Extracting the acetylene from the second cracking gas stream, wherein acetylene and/or synthesis gas are obtained;
   wherein a feed ratio of the hydrocarbon and oxidizing agent feeds to the acetylene synthesis reactor is controlled as such, that the oxygen ratio is constant, preferably, the control is (semi-)automatic;
   wherein the process is preferably the process according to any one of claims 1 to 7.

Fig. 1

Fig. 2

Europäisches
Patentamt

European
Patent Office

Office européen
des brevets

**EUROPEAN SEARCH REPORT**

Application Number

EP 24 16 5283

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 952 743 A (COME GUY-MARIE [FR]) 28 August 1990 (1990-08-28) * examples 1-4; claims 1 and 2 * | 1-11,13 | INV. C07C2/78 C07C11/24 C01B3/36 C25B1/04 |
| X | JP S61 97103 A (BRITISH PETROLEUM CO) 15 May 1986 (1986-05-15) * claim 1; tables 1-3 * | 1-11,13 | |
| X | GB 1 472 739 A (SHEVCHUK V; SYPYAK O) 4 May 1977 (1977-05-04) * examples 1-4; claim 1 * | 1-11,13 | |
| X | US 2 679 541 A (BERG CLYDE H O) 25 May 1954 (1954-05-25) * claim 1 * | 1-11,13 | |
| X | ZHANG SUISUI ET AL: "Life cycle assessment of acetylene production from calcium carbide and methane in China", JOURNAL OF CLEANER PRODUCTION, ELSEVIER, AMSTERDAM, NL, vol. 322, 16 September 2021 (2021-09-16), XP086822567, ISSN: 0959-6526, DOI: 10.1016/J.JCLEPRO.2021.129055 [retrieved on 2021-09-16] * the whole document * | 10,14,15 | TECHNICAL FIELDS SEARCHED (IPC) C07C C01B C25B |
| X | CN 219 849 538 U (CECEP ENGINEERING TECH RESEARCH INSTITUTE CO LTD) 20 October 2023 (2023-10-20) * example 1 * | 10 | |
| X | US 2021/061655 A1 (EL-HALWAGI MAHMOUD M [US] ET AL) 4 March 2021 (2021-03-04) * claim 1 * | 12 | |

-/--

The present search report has been drawn up for all claims

| Place of search | Date of completion of the search | Examiner |
|---|---|---|
| Munich | 4 October 2024 | Matés Valdivielso, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt
European
Patent Office
Office européen
des brevets

# EUROPEAN SEARCH REPORT

**Application Number**

EP 24 16 5283

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FR 3 110 917 A3 (AIR LIQUIDE [FR]) 3 December 2021 (2021-12-03) * claim 12 * | 12 | |
| X | US 11 111 195 B2 (ULTRA CLEAN ECOLENE INC [CA]) 7 September 2021 (2021-09-07) * claim 1 * | 12 | |
| X | US 2021/215095 A1 (HIROKANE TAKESHI [JP] ET AL) 15 July 2021 (2021-07-15) * examples 1-4; claim 1 * | 12 | |
| X | GB 2 618 418 A (JOHNSON MATTHEY DAVY TECHNOLOGIES LTD [GB]) 8 November 2023 (2023-11-08) * claims 1,7,20 * | 16-19 | |

**TECHNICAL FIELDS SEARCHED (IPC)**

The present search report has been drawn up for all claims

| Place of search | Date of completion of the search | Examiner |
|---|---|---|
| Munich | 4 October 2024 | Matés Valdivielso, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

EP 24 16 5283

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-19

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

The Search Division considers that the present European patent application does not comply with the requirements of unity of invention and relates to several inventions or groups of inventions, namely:

1. claims: 1-19

   Process for preparing acetylene and/or synthesis gas according to claim 1, as well as related products and uses thereof.
   - - -

2. claims: 20, 21

   Use of delta-18O value in oxygen and downstream compounds for tracing their origin, as well as related process.
   - - -

3. claim: 22

   Process for preparing acetylene and/or synthesis gas according to claim 22.
   - - -

## ANNEX TO THE EUROPEAN SEARCH REPORT
## ON EUROPEAN PATENT APPLICATION NO.

EP 24 16 5283

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-2024

| Patent document cited in search report | | Publication date | Patent family member(s) | | Publication date |
|---|---|---|---|---|---|
| US 4952743 | A | 28-08-1990 | AT | E74119 T1 | 15-04-1992 |
| | | | AU | 2580788 A | 15-06-1989 |
| | | | CA | 1338791 C | 10-12-1996 |
| | | | CN | 1042898 A | 13-06-1990 |
| | | | EP | 0319369 A1 | 07-06-1989 |
| | | | ES | 2030195 T3 | 16-10-1992 |
| | | | FR | 2624115 A1 | 09-06-1989 |
| | | | GR | 3004142 T3 | 31-03-1993 |
| | | | JP | H01186825 A | 26-07-1989 |
| | | | NO | 174743 B | 21-03-1994 |
| | | | NZ | 227038 A | 28-08-1990 |
| | | | US | 4952743 A | 28-08-1990 |
| | | | US | 5124134 A | 23-06-1992 |
| JP S6197103 | A | 15-05-1986 | AU | 4838185 A | 24-04-1986 |
| | | | CN | 85108595 A | 10-05-1986 |
| | | | DK | 478985 A | 19-04-1986 |
| | | | EP | 0178853 A2 | 23-04-1986 |
| | | | JP | S6197103 A | 15-05-1986 |
| | | | NZ | 213854 A | 29-04-1988 |
| | | | US | 4726913 A | 23-02-1988 |
| | | | ZA | 857807 B | 27-05-1987 |
| GB 1472739 | A | 04-05-1977 | NONE | | |
| US 2679541 | A | 25-05-1954 | NONE | | |
| CN 219849538 | U | 20-10-2023 | NONE | | |
| US 2021061655 | A1 | 04-03-2021 | US | 2021061655 A1 | 04-03-2021 |
| | | | WO | 2019147786 A1 | 01-08-2019 |
| FR 3110917 | A3 | 03-12-2021 | NONE | | |
| US 11111195 | B2 | 07-09-2021 | CA | 2980573 A1 | 17-07-2018 |
| | | | CA | 3076104 A1 | 04-04-2019 |
| | | | US | 2020283362 A1 | 10-09-2020 |
| | | | WO | 2019060988 A1 | 04-04-2019 |
| US 2021215095 | A1 | 15-07-2021 | CN | 111684048 A | 18-09-2020 |
| | | | JP | WO2019151461 A1 | 12-02-2021 |
| | | | KR | 20200118066 A | 14-10-2020 |
| | | | US | 2021215095 A1 | 15-07-2021 |
| | | | WO | 2019151461 A1 | 08-08-2019 |
| GB 2618418 | A | 08-11-2023 | CN | 118679137 A | 20-09-2024 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.** EP 24 16 5283

This annex lists the patent family members relating to the patent documents cited in  the above-mentioned European search report.
The members are as contained in the European Patent Office EDP file on
The European Patent Office is in no way liable for these particulars which are merely given for the purpose of information.

04-10-2024

| Patent document cited in search report | Publication date | Patent family member(s) | | Publication date |
|---|---|---|---|---|
| | | EP | 4496784 A1 | 29-01-2025 |
| | | GB | 2618418 A | 08-11-2023 |
| | | WO | 2023180683 A1 | 28-09-2023 |

For more details about this annex : see Official Journal of the European Patent Office, No. 12/82

EPO FORM P0459

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5824834 A
- EP 12171956 A
- EP 1989160 A
- US 9802875 B
- US 9580312 B
- US 20220107618 A1
- US 20220108327 A1

- US 20220108326 A1
- DE 875198
- DE 1051845
- DE 1057094
- DE 4422815
- WO 2015028539 A
- WO 2013186291 A

**Non-patent literature cited in the description**

- Acetylene. Ullmann's Encyclopedia of Industrial Chemistry. Wiley-VCH Verlag GmbH & Co. KGaA, 2012
- Acetylene from Hydrocarbons. **GANNON, R.E. et al.** Kirk-Othmer Encyclopedia of Chemical Technology. John Wiley & Sons Inc., 2000, vol. 1, 177-227
- **HESARI et al.** General design consideration of cryogenic air separation unit for esfahan steel company. *J. Gas Technology*, 2020, vol. 5 (1), 32-42
- *The Product Carbon Footprint Guideline for the Chemical Industry*, November 2022, https://www.tfs-initiative.com
- **H. OZCAN et al.** Recent advances, challenges, and prospects of electrochemical water-splitting technologies for net-zero transition. *Cleaner Chem. Eng.*, 2023, vol. 8, 100115, https://doi.org/10.1016/j.clce.2023.100115
- **J. BRAUNS** ; **T. TUREK**. *Processes*, 2020, vol. 8 (2), 248
- **S. KUMAR** ; **V. HIMABINDU**. *Material Science for Energy Technologies*, 2019, vol. 2, 4442-4454
- **H. A. MILLER et al.** *Sustainable Energy Fuels*, 2020, vol. 4, 2114-2133
- **K. KAMLUNGSUA et al.** *FUEL CELLS*, 2020, vol. 20 (6), 644-649
- **M. VICARI**. BASF and Acetylene - 70 Years of Reppe Chemistry - Long-Standing Reliability and Promising Future - and now, the only Natural Gas Based Clean Technology for Acetylene Production. *DGMK-Tagungsbericht*, February 2013, ISBN 978-3-941721-32-6, 99-102
- Acetylene. Ullmann's Encyclopedia of industrial chemistry. Wiley-VCH Verlag GmbH & Co. KGaA, 2012

- Acetylene. **P. PÄSSLER et al.** Ullmann's Encyclopedia of Industrial Chemistry.. Wiley-VCH Verlag, 2011
- Conversion of Methane to Acetylene. **V. GALVITA et al.** Methane Conversion Routes - Status and Prospects, Green Chemistry Series. Royal Society of Chemistry, 2024
- **BRIAN P. FLANNERY** ; **JAN W. MARES**. Resources for the Future (independent, nonprofit research institution, Washington, DC). *The Greenhouse Gas Index for Products in 39 Industrial Sectors, Sectoral Modules*, September 2022, https://www.rff.org/publications/working-papers/the-greenhouse-gas-index-for-products-in-39-industrial-sectors
- **FERNANDEZ-GONZALEZ et al.** CO2 electroreduction: Sustainability analysis of the renewable synthetic natural gas. *Int. J. of Greenhouse Gas Control*, 2022, vol. 114, 103549, https://doi.org/10.1016/j.ijggc.2021.103549
- The mean CF of NG in Europe is estimated as 0.593 kg CO2e/kg NG and varies from a representative range depending on the country and source within 0.166 to 0.917 kg CO2e/kg NG. *Swiss Centre for Life Cycle Inventories*, 2020, 4
- **FLANNERY et al.** WP22-16 M22; NAICS CODE 325120, 3-4
- **BELAISSAOUIA et al.** Energy Efficiency of Oxygen Enriched Air Production Technologies: Cry-ogeny vs Membranes. *Energy Procedia*, 2014, vol. 63, 497-503
- **NEL ASA**. *Alkaline and Proton PEM Electrolysers*

- Servomex Total Solution for Gas Analysis on Cryogenic Air Separation Plants. *ASU oxygen purity ranges: SERVOMEX brochure AB15 Rev.0 04/11 E*, January 2024, https://www.dastec-srl.com.uy/uploads/aplicacion-downloads/migracion/2/104.pdf?v154
- **ZENG, K.** ; **ZHANG, D.** Recent progress in alkaline water electrolysis for hydrogen production and applications. *Progress in Energy and Combustion Science: an international review journal*, 2010, vol. 36, 307-326, https://doi.org/10.1016/j.pecs.2009.11.002
- **VINCENT KNOP**. Part load operation of alkaline electrolysers. *A World Of Energy*, 12 March 2023, https://www.awoe.net/Water-Electrolysis-Alkaline-Part-Load.html

- *Fuel*, 2022, vol. 317, 123452, https://doi.org/10.1016/j.fuel.2022.123452
- **MARTINS et al.** Stable isotopes of oxygen and hydrogen in water: analytical method evaluation and the determination of $\delta$ 18O and $\delta$ 2H on a control sample. *Quim. Nova*, 2023, vol. 46 (4), 343-350, http://dx.doi.org/10.21577/0100-4042.20230021
- **WASSENAAR et al.** An On-Line Technique for the Determination of the $\delta$ 18O and $\delta$ 17O of Gaseous and Dissolved Oxygen. *Anal. Chem.*, 1999, vol. 71, 4965-4968
- **PATHIRANA et al.** An analytical system for studying the stable isotopes of carbon monoxide using continuous flow-isotope ratio mass spectrometry (CF-IRMS). *Atmos. Meas. Tech. Discuss.*, 2015, vol. 8, 2067-2092